# EUROPEAN PATENT APPLICATION

(11) **EP 4 223 313 A1**
(43) Date of publication of application: **09.08.2023**
(21) Application number: 21874572.7
(22) Date of filing: 30.09.2021
(51) Int. Cl.: A61K 45/06, A61K 9/10, A61K 47/04, A61K 47/12, A61K 47/18, A61K 47/22, A61P 35/00, A61P 17/00, A61P 31/00, A61P 5/00

(54) **PHARMACEUTICAL COMPOSITION COMPRISING ACID-BASE NEUTRALIZATION COMBINATION AND APPLICATION THEREOF**

(30) Priority: 30.09.2020 CN 202011064448; 30.09.2020 CN 202011059746; 18.02.2021 WO PCT/CN2021/076749; 22.02.2021 WO PCT/CN2021/077290; 03.03.2021 WO PCT/CN2021/078914
(71) Applicant: Chengdu Kuachangaopu Medical Technology Co., Ltd., Chengdu, Sichuan 610000 (CN); Kuachang Inc., Anaheim, CA 92804 (US)
(72) Inventor: ZOU, Fanglin, Chengdu, Sichuan 610041 (CN); ZOU, Lichang, Chengdu, Sichuan 610041 (CN); WANG, Jianxia, Chengdu, Sichuan 610041 (CN)
(74) Representative: ABG Intellectual Property Law, S.L.
(86) International application number: PCT/CN2021/122041
(87) International publication number: WO 2022/068918

(57) **Abstract**

The disclosure of the present application relates to a use of an acid-base neutralization combination as a local active ingredient in manufacturing a local medicine for the treatment of a local lesion disease, and a local pharmaceutical composition comprising the combination, and a method for treating a local lesion disease.

## Description

### Technical Field

The disclosure of the present application relates to a use of an acid-base neutralization combination as a local active ingredient in manufacturing a local medicament for the treatment of a local lesion disease, and a local pharmaceutical composition comprising the combination, as well as a method for treating a local lesion disease.

### Background Art

Supported by a large amount of research work, solid tumors are often used as research models for local lesion diseases, especially refractory local lesion diseases. Solid tumor is a tumor disease with a tumor body symptom. A tumor body is a characteristic pathological tissue containing tumor cells. Taking a pancreatic cancer tumor body as an example, pancreatic cancer cells account for only about 30% of the volume. It can be seen that in addition to tumor cells, there are often a larger number of other components (sometimes called a microenvironment of tumor cells) in a tumor body tissue, including various other cells, various intercellular substances, and various tubes.

Local administration has an advantage of physically targeting of drugs. Therefore, it was once believed that topical administration of cytotoxic drugs can increase intratumoral concentration of the drugs, thereby improving their efficacy. However, topical administration of cytotoxic drugs has not shown significant improvement in efficacy. Merely increasing the intratumoral concentration does not seem to be able to significantly improve efficiency of the drugs in targeting cancer cells in intratumoral tissues. In addition to resorting to a delayed release form, cytotoxic drugs are still almost administered systemically in clinical practice. Chemical ablation agents (high-purity ethanol, high-concentration acid and base) are not characterized by cell destruction but tissue destruction. Compared with cytotoxic drugs, they have almost no systemic therapeutic effect of targeting cancer cells, but often show local therapeutic effects. In the case of acid-base chemical ablation agents, their extreme acidity/alkalinity is considered to be the basis for their action. However, they are usually strong destructive agents that cannot sufficiently recognize a target tissue from other tissues. This makes their local stimulation strong. Their low efficiency and high irritation make their practical application limited. Therefore, chemical ablation agents have gradually faded out of clinic treatment of malignant solid tumors in the last decade. In fact, there are almost no topical drugs with both high local safety and high local efficacy in clinical practice.

Therefore, there is still a need to develop new drugs, especially topical drugs, for treating a local lesion disease such as a solid tumor, to meet various clinical needs that cannot be met in the prior art. In fact, it is also urgently needed for prevention and treatment of other local lesion diseases, especially refractory local lesion diseases.

### Summary of the invention

The object of the present invention is to provide a local medicine for preventing and treating a local lesion disease, especially a refractory local lesion disease. More specifically, the object of the present invention is to provide a local drug that is superior in specificity, effectiveness and safety to chemical ablation agents in the prior art (acidifying agents or basifying agents) or a pharmaceutical compositions comprising thereof.

According to an aspect of the present invention, it provides a use of an acid-base neutralization combination as a local active ingredient in manufacturing a local pharmaceutical composition for the treatment of a local lesion disease, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

According to another aspect of the present invention, it provides a local pharmaceutical composition for the treatment of a local lesion disease, comprising an acid-base neutralization combination as a local active ingredient, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

According to a further aspect of the present invention, it provides a local pharmaceutical composition for the treatment of a local lesion disease, comprising an acid-base neutralization combination as a local active ingredient and a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

In some embodiments, the composition of the present invention further comprises a pharmaceutically acceptable carrier, such as an aqueous carrier or an alcoholic carrier, preferably water. In some embodiments, the composition of the present invention can be prepared as dosage form of injection formulation. The injection formulation comprises liquid injection formulation, powder formulation for injection.

According to a further aspect of the present invention, it provides a method for treating a local lesion disease, comprising administering to a local lesion region of an individual in need thereof a therapeutically effective amount of a pharmaceutical composition that can cause tissue necrosis in a target region, wherein the pharmaceutical composition comprises an acid-base neutralization combination capable of acting as a local active ingredient and optionally a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

The embodiments according to the present invention have the following advantages over the acid-base chemical ablation agents of the prior art: higher specificity, effectiveness and safety.

The embodiments according to the present invention have the following advantages over the pharmaceutical compositions in the prior art comprising acid-base chemical ablation agents: higher specificity, effectiveness and safety.

The embodiments according to the present invention have the following advantages over those in the prior art for the treatment of a local lesion disease: compared with a cytotoxic drug of prior art, it shows almost non-toxic systemic safety and significantly higher efficacy on a local lesion disease; compared with a molecular targeted drug, it shows less stringent screening of indications, and has great potential for rapidly growing tumors, large tumors, and hypovascular tumors. The use and composition of the present invention are also not troubled by the drug resistance problems encountered by prior art cytotoxic drugs and existing molecular targeted drugs. In addition, the use and composition are easy to prepare and low in cost, which is especially helpful to provide safe and effective treatment for general population who cannot afford high expenses.

### Detailed Description of the Embodiments

As taught in the prior art, elevated pH in local lesions is the pharmacological basis for the use of a basifying agent (e.g., sodium hydroxide) as a chemical ablation agent. The inventors of the present invention, in conducting chemical ablation studies using basifying agents, mixing sodium bicarbonate, which has a much lower pH, as a pH neutralizing agent, with sodium hydroxide in order to use this acid-base neutralization composition (3% sodium hydroxide/7% sodium bicarbonate) as a negative control. As presumed according to the prior art disclosures, the pharmaceutical efficacy of sodium hydroxide solution would decrease with pH decreased (e.g., from pH 12 to 10.0) after it was neutralized by addition of sodium bicarbonate. Unexpectedly, however, the low pH composition (an acid-base neutralization composition) showed significantly higher tumor inhibition rate than the high pH sodium hydroxide (e.g., the tumor inhibition rate increases from 43% to 68%). Thus, the inventors of the present invention investigated pharmaceutically acceptable acid-base neutralization compositions, and further investigated pharmaceutical combinations comprising the acid-base neutralization combination and its local synergists, and uses thereof.

Accordingly, according to an aspect of the present invention, it provides a use of an acid-base neutralization combination as a local active ingredient in manufacturing a local pharmaceutical composition for the treatment of a local lesion disease, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases. In an embodiment, the local pharmaceutical composition further comprises a local synergist of the acid-base neutralization combination.

In one embodiment, the local activity provided by the acid-base neutralization combination comprises an effective inter-tissue penetration effect, preferably a maximum inter-tissue penetration effect.

In one embodiment, the local active component comprises a component having an effective inter-tissue penetration effect, preferably a maximum inter-tissue penetration effect.

In one embodiment, the pharmaceutical composition is so prepared as to make the acid-base neutralization combination provide the local activity: the acidifying agent and its pH neutralizing agent, or the basifying agent and its pH neutralizing agent are administered at an amount ratio (w/w) (W _{basifying agent}/W _{neutralizing agent}, or W _{acidifying agent}/W _{neutralizing agent}) of (0.5-35)/(1-35), preferably (1.5-35)/(1-35).

In one embodiment, the pharmaceutical composition is so prepared as to make the acid-base neutralization combination provide the local activity: the acidifying agent or the basifying agent is administered at a concentration of ≥0.5%, preferably ≥0.75% or ≥2.5%, and the pH neutralizing agent should be administered at such a concentration that the administration pH value of the pharmaceutical composition is closer to neutral than the pH of a single drug having the same concentration of the acidifying agent or the basifying agent, and the absolute value of the difference between the two pHs (|administration pH of the pharmaceutical composition - pH of the single drug having the same concentration of the acidifying agent or the basifying agent|) is ≥ 0.25, ≥ 0.5, or ≥ 1.0.

According to another aspect of the present invention, it provides a local pharmaceutical composition for the treatment of a local lesion disease, comprising an acid-base neutralization combination as a local active ingredient, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance can make the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

In one embodiment, the pharmaceutical composition is so composed as to make the acid-base neutralization combination provide the local activity: the acidifying agent and its pH neutralizing agent, or the basifying agent and its pH neutralizing agent are administered at an amount ratio (w/w) (W _{basifying agent}/W _{neutralizing agent}, or W _{acidifying agent}/W _{neutralizing agent}) of (0.5-35)/(1-35), preferably (1.5-35)/(1-35).

In one embodiment, the pharmaceutical composition is so composed as to make the acid-base neutralization combination provide the local activity: the acidifying agent or the basifying agent is administered at a concentration of ≥0.5%, preferably ≥0.75% or ≥2.5%, and the pH neutralizing agent should be administered at such a concentration that the administration pH value of the pharmaceutical composition is closer to neutral than the pH of a single drug having the same concentration of the acidifying agent or the basifying agent, and the absolute value of the difference between the two pHs (|administration pH of the pharmaceutical composition - pH of the single drug having the same concentration of the acidifying agent or the basifying agent|) is ≥ 0.25, ≥ 0.5, or ≥ 1.0.

According to a further aspect of the present invention, it provides a local pharmaceutical composition for the treatment of a local lesion disease, comprising an acid-base neutralization combination as a local active ingredient and a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralization, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

In one embodiment, the pharmaceutical composition is so composed as to make the acid-base neutralization combination provide the local activity: the acidifying agent and its pH neutralizing agent, or the basifying agent and its pH neutralizing agent are administered at an amount ratio (w/w) (W _{basifying agent}/W _{neutralizing agent}, or W _{acidifying agent}/W _{neutralizing agent}) of (0.5-35)/(1-35), preferably (1.5-35)/(1-35). In one embodiment, the pharmaceutical composition is so composed as to meet the requirements of the acid-base neutralization combination and the local synergist as a local active component providing local synergistic effect: the administration amount ratio (w/w) of the acid-base neutralization combination to the local synergist (w/w) (W _{local synergis}/W _{acid-base neutralization combination}) is (0.1-45)/(1.5-45).

In one embodiment, the pharmaceutical composition is so composed as to make the acid-base neutralization combination provide the local activity: the acidifying agent or the basifying agent is administered at a concentration of ≥0.5%, preferably ≥0.75% or ≥2.5%, and the pH neutralizing agent should be administered at such a concentration that the administration pH value of the pharmaceutical composition is closer to neutral than the pH of a single drug having the same concentration of the acidifying agent or the basifying agent, and the absolute value of the difference between the two pHs (|administration pH of the pharmaceutical composition - pH of the single drug having the same concentration of the acidifying agent or the basifying agent|) is ≥ 0.25, ≥ 0.5, or ≥ 1.0.

According to a further aspect of the present invention, it provides a method for treating a local lesion disease, comprising administering to a local lesion region of an individual in need thereof a therapeutically effective amount of a pharmaceutical composition that can cause tissue necrosis in the target region, wherein the pharmaceutical composition comprises an acid-base neutralization combination capable of acting as a local active ingredient and optionally a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralize, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

In one embodiment, the pharmaceutical composition is so administered as to make the acid-base neutralization combination provide the local activity: the acidifying agent or the basifying agent is administered at a concentration of ≥0.5%, preferably ≥0.75% or ≥2.5%, and the pH neutralizing agent should be administered at such a concentration that the administration pH value of the pharmaceutical composition tend to be neutral than the pH of a single drug having the same concentration of the acidifying agent or the basifying agent, and the absolute value of the difference between the two pHs (|administration pH of the pharmaceutical composition - pH of the single drug having the same concentration of the acidifying agent or the basifying agent|) is ≥ 0.25, ≥ 0.5, or ≥ 1.0.

In a further aspect of the present application disclosure, it provides a device for treating a local lesion disease comprising the local pharmaceutical composition comprising the acid-base neutralization combination as defined according to the present application disclosure.

In the scope of the present invention, the term "pharmaceutical composition" (or pharmaceutical combination) as used herein refers to a mixture of more than one drug uniformly distributed in the same formulation. The term "local pharmaceutical composition" refers to a pharmaceutical composition of a plurality of active components that can be administered locally and adequately mixed at desired concentrations or concentration ratios and entered into the target region; preferably the "local pharmaceutical composition" does not comprise additives such as sodium chloride (isotonic regulators) and the like that are commonly used for non-local medicines.

In the scope of the present invention, the term "acidifying agent (or basifying agent)" refers to the most alkaline substance or the most acidic substance in the composition. The term "pH neutralizing agent (or neutralizing agent)" refers to an acidic substance or an alkaline substance that increases the pH of an acidifying agent (or decreases the pH of a basifying agent), thus makes the pH of the composition tend to be neutral (pH about 7.0). The term "conventional active component" refers to a pharmaceutical component that can provide effective activity when administered systemically. The term "pH adjusting agent" refers to a pharmaceutical component that provides primarily pH adjustment rather than a pharmaceutical activity (e.g., anti-pathogen activity) when administered systemically.

The pharmacological effects of the pharmaceutical compositions disclosed in the present application comprise synergistic effect and optionally other pharmacological effects. In one embodiment, the synergistic effects provided by an acid-base neutralization combination itself or by a composition comprising the acid-base neutralization combination and a co-administration substance are characterized by one or more of the following: the synergistic effects include safety synergistic effects (e.g., synergistic effects that result in reduced local irritation, synergistic effects that result in reduced doses of a required acidifying agent (or a required basifying agent)), local synergistic effects (shown as short-term synergistic effects), inter-tissue effective synergistic penetration (shown as medium-term synergistic effects).

In the scope of the present disclosure, unless otherwise specified, the term "a concentration (or an amount ratio) at which concentration (or amount ratio) entering the target region" (or a concentration or an amount ratio entering the target region) means a concentration (or an amount ratio) at which a particular component begins to contact the target region, the concentration (or the amount ratio) is equivalent to the administration concentration (or administration amount ratio) in the local (target region) administration of the present invention, and is not equivalent to the administration concentration (or administration amount ratio) in conventional (systemic) administration.

In the scope of the present disclosure, the term "target region" as used herein refers to a target site of administration, such as an adjacent area, interface, interior (preferably the internal part) of a local lesion, and the like. The term "local activity" refers to the pharmacological activity of the drug preferentially within the tissue of the local target region rather than within the pathogen (e.g., within tumor cells) and includes, for example, the local effect of a single drug and the local co-administration effect of a co-administration drug. The term "local effect (or local co-administration effect)" is distinguished from a conventional effect (or conventional co-administration effect), the latter refers to a pharmacological effect (or a co-administration effect) produced by a drug that is absorbed into the bloodstream to form drug-carrying blood after a conventional administration (e.g., intravenous injection, intracavitary injection, subcutaneous administration, mucosal administration, etc.) and enters the target region, whereas the former refers to a pharmacological effect (or co-administration effect) of a drug preferentially produced in a target region after local administration to the target region, and the local effect (or local co-administration effect) includes a chemical effect (or co-administration chemical effect) preferentially targeting the target region tissue. The term "local chemical effect (or local co-administration chemical effect)" refers to a chemical effect (or co-administration chemical effect) of a drug occurring preferentially in the local tissue rather than in the pathogen in that tissue. The term "local active ingredient" is used to refer to a pharmaceutical ingredient that can provide a pharmacological activity, including effective local activity, when used locally alone or in combination.

In one embodiment, the local synergistic effect is preferably a synergistic effect that occurs preferentially within a local tissue rather than within a pathogen in that tissue. In one embodiment, the local synergistic effect comprises a local chemically synergistic effect and optionally other pharmacological effects. In one embodiment, the pharmaceutical composition is a locally synergistic composition. In one embodiment, the pH neutralizing agent and the acidifying agent or the basifying agent are locally synergistic in the same solution. In one embodiment, the local synergist and the acid-base neutralization combination are locally synergistic in the same solution. In the scope of the present disclosure, the term "synergistic effect" means that co-administration of the active components shows a desired pharmaceutical efficacy that is higher than that of any active component administered alone (e.g., a pharmaceutical efficacy having an actual/expected ratio q >1.0 designated in Examples of the present invention). The term "local synergistic effect" refers to one primarily shown as synergistic effect of local activity. The term "local synergist" refers to a pharmaceutical component that can produce a locally synergistic effect by co-administration, in particularly by co-administration with the pH neutralization combination of the present invention. Within the scope of the present application, systemic co-administration of a local synergist with a specific drug (e.g., the pH neutralization combination) either does not produce a synergistic effect or produces a synergistic effect that is not a local synergistic effect.

In the scope of the present disclosure, the term "therapeutically effective amount" refers to the dose of a drug that is used to treat a disease (e.g., a tumor) and achieves an effective effect (e.g., reduction or/and relief of disease symptoms).

The pharmaceutical composition of the present disclosure is so prepared, composed or administered that they meet the need for the local synergistic effect to cause more efficient necrosis, preferably effective necrosis, of the target region tissue. In one embodiment, the local synergistic effect is a pharmacodynamic synergy, such as using equal doses of the acidifying agent or the basifying agents to provide higher therapeutic efficacy. In one embodiment, the local synergistic effect is a safety synergy, such as using minimal net acid or net base (or minimal disturbance of acid-base balance in vivo) to produce the same therapeutic efficacy. In one embodiment, the tissue necrosis comprises direct tissue necrosis and, optionally indirect tissue necrosis. In one embodiment, the direct tissue necrosis is so intense that the tissue is chemically ablated.

In the scope of the present disclosure, the term "indirect tissue necrosis" as used to refer to tissue necrosis caused by the inhibition of the proliferation of pathogens (e.g., cells, viruses, or bacteria) in the target region tissue or by the effect of stimulation on an individual's immune system, wherein the former, for example, cytotoxic drugs, by inhibiting cancer cells, may eventually lead to necrosis of the tissue in which the cancer cells are located, and the latter, for example, antitumor vaccines, by stimulating the immune system to attack cancer cells, may eventually lead to necrosis of the tissues in which the cancer cells are located. Therefore, the indirect necrosis can be achieved either by systemic or local administration. The term "direct tissue necrosis" as used to refer to tissue necrosis that results from the direct administration of tissue-damaging factors to a target region, which is not dependent on indirect tissue necrosis, wherein the tissue-damaging factors include, for example, ablation parts of physical ablation instruments (e.g., warming needles), conventional chemical ablation agents, etc.

In the scope of the present invention, the term "ablation" refers to local effects by in situ treatment are so strong that the target tissue occurs tissue necrosis that essentially loses its original physiological function (inactivation). Tissue necrosis includes coagulative necrosis, liquefied necrosis, fibrinoid necrosis, and so on. Tumor tissue necrosis can result in morphological changes (volume reduction) of the tumor tissue, which ultimately results in increased tumor inhibition rate. The term "chemical ablation" refers to an ablation that is primarily chemical rather than immunologic. The term "chemical ablation agent" refers to drugs that show ablative effects only when administered locally.

In the scope of the present invention, unless otherwise stated, the term "concentration" refers to the weight percent concentration (w/v) of a specified component in the unit volume of a local pharmaceutical composition. The term "local administration amount ratio" (or administration amount ratio) refers to the weight ratio of the two specified components when the drug is administered locally, which may be the weight ratio of the specified components at the drug contact target region (e.g., injection needle hole or perfusion tube outlet). The term "local administration concentration" (or administration concentration) refers to the concentration of the specified component at the time of local administration of the drug, which may be the concentration of the specified component at the drug contact target region (e.g., injection needle hole or perfusion tube outlet). The term "local administration pH" (or administration pH) refers to the pH of the drug at the time of local administration, which may be the pH of the drug at the contact target region (e.g., injection needle hole or perfusion tube outlet).

In one embodiment, the synergistic composition is a composition having synergistic safety. In one embodiment, the synergistic composition is a composition having synergistic pharmaceutical efficacy.

In one embodiment, the pharmaceutical composition has an administration pH of 7.5±4.5, preferably 10.0±2.0 or 4.0±2. In one embodiment, the pharmaceutical composition has an administration pH of 8.5-11.5, 9.5-11.5, or 10.0-11.0. In one embodiment, the pharmaceutical composition has an administration pH of 3.0-5.0, or 3.5-4.5.

In one embodiment:
the basifying agent is a strong base, and the pharmaceutical composition is so prepared, composed or administered as to make the acid-base neutralization combination provide the local activity: the strong base is administered at a concentration (w/v) of ≥0.5%, preferably ≥0.75% or ≥1%; of 0.5-10%, preferably 0.75-10% or 1-10%; or
the basifying agent is a weak base, and the pharmaceutical composition is so prepared, composed or administered as to make the acid-base neutralization combination provide the local activity: the weak base is administered at a concentration (w/v) of ≥2.5%, preferably ≥3.0% or ≥5%; of 2.5-35%, preferably 3.0-35% or 5-35%; or
the acidifying agent is a strong acid, and the pharmaceutical composition is so prepared, composed or administered as to make the acid-base neutralization combination provide the local activity: the strong acid is administered at a concentration (w/v) of ≥ 0.5%, preferably ≥ 0.75% or ≥ 1%; of 0.5-10%, preferably 0.75-10% or 1-10%; or
the acidifying agent is a weak acid, and the pharmaceutical composition is so prepared, composed or administered as to make the acid-base neutralization combination provide the local activity: the weak acid is administered at a concentration (w/w) of ≥2.5%, preferably ≥3.0% or ≥5%, or of 2.5-20%, preferably 3.0-20% or 5-20%.

In one embodiment, the pharmaceutical composition is so prepared, composed or administered as to make the acid-base neutralization combination provide the local activity: the pH neutralizing agent is selected from a group consisting of one or more of: a weak base, a salt compounded with a strong acid and/or a strong base, an alkali metal salt of a weak organic acid, a weak acid, and the pH neutralizing agent is administered at a concentration of ≥1%, preferably 2-35%.

In one embodiment, the basifying agent is one or more of strong bases, the pH neutralizing agent is selected from a group consisting of one or more of: a weak base, a salt compounded with a strong acid and a strong base, an alkali metal salt of weak organic acid, and a weak acid, and the pharmaceutical composition is so prepared, composed or administered as to make the acid-base neutralization combination provide the local activity: the strong base is administered at a concentration of ≥0.5%, preferably ≥0.75% or ≥1%; of 0.5-10%, preferably 0.75-10% or 1-10%, and the pH neutralizing agent is administered at a concentration of ≥1%, preferably 2-35%.

In one embodiment, the basifying agent is one or more of weak bases, the pH neutralizing agent is selected from one or more of a group consisting of: other weak bases, a salt compounded with a strong acid and/or a strong base, an alkali metal salt of weak organic acid, and a weak acid, and the pharmaceutical composition is so prepared, composed or administered as to make the acid-base neutralization combination provide the local activity: the weak base is administered at a concentration of ≥2.5%, preferably ≥3.0% or ≥5%; of 2.5-35%, preferably 3.0-35% or 5-35%, and the pH neutralizing agent is administered at a concentration of ≥1%, preferably 2-35%.

In one embodiment, the acidifying agent is one or more of strong acids, the pH neutralizing agent is selected from a group consisting of one or more of: a weak base, a salt compounded with a strong acid and/or a strong base, an alkali metal salt of weak organic acid, and a weak acid; and the pharmaceutical composition is so prepared, composed or administered as to make the acid-base neutralization combination provide the local activity: the strong acid is administered at a concentration of ≥ 0.5%, preferably ≥ 0.75% or ≥ 1%; of 0.5-10%, preferably 0.75-10% or 1-10%, and the pH neutralizing agent is administered at a concentration of ≥ 1%, preferably 2-35%.

In one embodiment, the acidifying agent is one or more of weak acids, the pH neutralizing agent is one or more selected from a group consisting of: a weak base, a salt compounded with a strong acid and/or a strong base, an alkali metal salt of weak organic acid, and other weak acids; and the pharmaceutical composition is so prepared, composed or administered as to make the acid-base neutralization combination provide the local activity: the weak acid is administered at a concentration of ≥ 2.5%, preferably 2.5-20%, preferably 3.0-20% or 5-20% ≥ 0.75%, and the pH neutralizing agent is administered at a concentration of ≥ 1%, preferably 2-35%.

In one embodiment, the concentration of the strong base in the pharmaceutical composition is ≥0.5%, preferably ≥0.75% or ≥1%, or is 0.5-10%, preferably 0.75-10% or 1-10%.

In one embodiment, the concentration of the weak base in the pharmaceutical composition is ≥2.5%, preferably ≥3.0% or ≥5%, or is 2.5-35%, preferably 3.0-35% or 5-35%.

In one embodiment, the concentration of the strong acid in the pharmaceutical composition is ≥0.5%, preferably ≥0.75% or ≥1%, or is 0.5-10%, preferably 0.75-10% or 1-10%.

In one embodiment, the concentration of the weak acid in the pharmaceutical composition is ≥2.5%, preferably ≥3.0% or ≥5%, or is 2.5-20%, preferably 3.0-20% or ≥5-20%.

In one embodiment, the basifying agent is selected from a strong base, and the pharmaceutical composition has a pH of 10.0±2.0 or 10.5±1.0.

In one embodiment, the basifying agent is selected from a weak base, and the pharmaceutical composition has a pH of 8.0±1.0 or 8.5±0.5.

In one embodiment, the acidifying agent is selected from a weak acid, and the pharmaceutical composition has a pH of 4.0±1.0 or 4.0±0.5.

In one embodiment, the acidifying agent is selected from a strong acid, and the pharmaceutical composition has a pH of 3.0±1.0 or 3.0±0.5.

In one embodiment, addition of the pH neutralizing agent makes the pH of the pharmaceutical composition tend to neutralize, and increases the buffering capacity of the pharmaceutical composition.

In one embodiment, the pharmaceutical composition has a buffering capacity of >0.01mol·L⁻¹ ·pH⁻¹, preferably 0.015-0.45mol·L⁻¹ ·pH⁻¹.

In one embodiment, the buffering capacity of the pharmaceutical composition is preferably ≥0.04mol·L⁻¹ ·pH⁻¹, more preferably ≥0.05mol·L⁻¹ ·pH⁻¹.

In one embodiment, the pharmaceutical composition has a buffering capacity of 0.04-0.45mol·L⁻¹ ·pH⁻¹, more preferably 0.05-0.45mol·L⁻¹ ·pH⁻¹.

In the scope of the present invention, the term "buffering capacity" (also known as buffering index) refers to the amount (e.g., x mol) of a monobasic strong acid (e.g., hydrochloric acid) or a monobasic strong base (e.g., sodium hydroxide) that needs to be added when pH of a unit volume (e.g., 1 L) of a pharmaceutical composition changes 1 unit, and is expressed as mol·L⁻¹ ·pH⁻¹.

In the scope of the present invention, the pH neutralizing agent is other than the acidifying agent or the basifying agent and makes the pH of the pharmaceutical composition more neutral, which is one or more selected from a group consisting of a strong base, a weak base, a salt compounded with a strong acid or a strong base, an alkali metal salt of weak organic acid, a weak acid, and a strong acid. In the pharmaceutical composition, the concentration of the pH neutralizing agent can be ≥1%, preferably 2-35%.

In the scope of the present invention, "the pH of the acidifying agent or the basifying agent tends to neutralize" means: when the acidifying agent or the basifying agent is selected from strong bases or weak bases, the difference between the pH of the acidifying agent or the basifying agent and the pH of the composition is ≥ 0.25; when the acidifying agent or the basifying agent is f selected rom strong acids or weak acids, the difference between the pH of the composition and the pH of the acidifying agent or the basifying agent is ≥ 0.25. The addition of the pH neutralizing agent can also result in an increase in the buffering capacity of the composition after the pH has been changed.

In the scope of the present invention, the pH neutralizing agent is preferably selected from water-soluble pharmaceutical excipient compounds and/or water-soluble auxiliary pharmaceutical compounds. In one embodiment, the concentration of the pH neutralizing agent can be ≥2% or ≥3%, preferably 3-35%.

In the scope of the present invention, the weak base is one or more selected from a group consisting of alkali inorganic salt of polybasic weak acid, acid inorganic salt of polybasic weak acid, nitrogen-containing weak base.

In the scope of the present invention, the term "acid inorganic salt of polybasic weak acid" refers to a polybasic weak acid inorganic salt capable of ionizing hydrogen ions in water. The acid inorganic salt of polybasic weak acid as an acidifying agent or a basifying agent is preferably the one whose 0.01M aqueous solution is alkaline, preferably pH ≥ 8.0 (e.g. disodium hydrogen phosphate, sodium bicarbonate, potassium bicarbonate). The term "alkali inorganic salt of polybasic weak acid" refers to a polybasic weak acid inorganic salt that cannot ionize hydrogen ions in water, and includes a positive salt of a polybasic weak acid-strong base inorganic salt. The term "nitrogen-containing weak base" refers to a weakly basic compound containing nitrogen element, where the weakly basic compound is preferably selected from weakly basic compounds with water solubility (w/w) of ≥2%.

In one embodiment, the compositions include, for example, the compositions listed in Table 1 below.

**Table 1**

| Class | Basic components of the composition | | Specific examples of the composition |
|---|---|---|---|
| | acidifying agent or basifying agent | pH neutralizing agent | |
| I | strong base | alkali inorganic salt of polybasic weak acid | sodium carbonate-sodium hydroxide, borax-sodium hydroxide, sodium carbonate-potassium hydroxide |
| I | strong base | nitrogen-containing weak base | triethanolamine-sodium hydroxide, 2-aminoethanol-sodium hydroxide |
| I | strong base | acid inorganic salt of polybasic weak acid | sodium bicarbonate-sodium hydroxide, sodium dihydrogen phosphate-sodium hydroxide, sodium dihydrogen phosphate-potassium hydroxide, potassium dihydrogen phosphate-sodium hydroxide, potassium hydrogen phthalate-sodium |
| | | | hydroxide, disodium hydrogen phosphate-sodium hydroxide |
| I | strong base | salts compounded with strong acid and strong base | potassium chloride-sodium hydroxide, sodium chloride-potassium hydroxide, potassium iodide-sodium hydroxide |
| I | strong base | alkali metal salt of weak organic acid | sodium acetate-sodium hydroxide, sodium lactate-sodium hydroxide, sodium citrate-sodium hydroxide, sodium 2-hydroxypropane-1,2,3-tricarboxylate-sodiu m hydroxide |
| I | strong base | weak acid | phthalic acid-sodium hydroxide |
| I | strong base | alkali inorganic salt of polybasic weak acid and others | sodium acetate-sodium carbonate-sodium hydroxide, sodium citrate-sodium carbonate-sodium hydroxide, sodium 2-hydroxypropane-1,2,3-tricarboxylate-sodiu m carbonate-sodium hydroxide |
| I | strong base | acid inorganic salt of polybasic weak acid and others | sodium bicarbonate-sodium carbonate-sodium hydroxide, sodiumu dihydrogen phosphate-disodium hydrogen phosphate-sodium hydroxide, sodium dihydrogen phosphate-sodium carbonate-potassium hydroxide, sodium acetate-sodium bicarbonate-sodium hydroxide, sodium citrate-sodium bicarbonate-sodium hydroxide, sodium 2-hydroxypropane-1,2,3-tricarboxylate-sodiu m bicarbonate-sodium hydroxide |
| II | weak base (alkali inorganic salt of polybasic weak acid) | nitrogen-containing weak base | triethanolamine-sodium carbonate, 2-aminoethanol-sodium carbonate |
| II | weak base (alkali inorganic salt of polybasic weak acid) | acid inorganic salt of polybasic weak acid | sodium bicarbonate-sodium carbonate, sodium dihydrogen phosphate-disodium hydrogen phosphate, disodium hydrogen phosphate-potassium dihydrogen phosphate, sodium dihydrogen phosphate-sodium carbonate |
| II | weak base | alkali metal salt of | sodium acetate-sodium carbonate, sodium |
| | (alkali inorganic salt of polybasic weak acid) | weak organic acid | lactate-sodium carbonate, sodium citrate-sodium carbonate, sodium 2-hydroxypropane-1,2,3-tricarboxylate-sodiu m carbonate |
| II | weak base (alkali inorganic salt of polybasic weak acid) | weak acid or/and strong acid | borax-hydrochloric acid, boric acid-borax, carbonic acid-sodium carbonate, phosphoric acid-disodium hydrogen phosphate, acetic acid-sodium carbonate |
| II | weak base (acid inorganic salt of polybasic weak acid) | nitrogen-containing weak base | triethanolamine-sodium bicarbonate, 2-aminoethanol-sodium bicarbonate |
| II | weak base (acid inorganic salt of polybasic weak acid) | alkali metal salt of weak organic acid | sodium acetate-sodium bicarbonate, sodium lactate-sodium bicarbonate, sodium citrate-sodium bicarbonate, sodium citratedisodium hydrogen phosphate |
| II | weak base (acid inorganic salt of polybasic weak acid) | weak acid or/and strong acid | carbonic acid-sodium bicarbonate, phosphoric acid-disodium hydrogen phosphate, acetic acid-sodium bicarbonate |
| II | weak base (nitrogen-conta ining weak base) | acid inorganic salt of polybasic weak acid | sodium bicarbonate-aqueous ammonia, sodium dihydrogen phosphate-aqueous ammonia, disodium hydrogen phosphate-aqueous ammonia, sodium dihydrogen phosphate-aqueous ammonia |
| II | weak base (alkali inorganic salt of polybasic weak acid) | acid inorganic salt of polybasic weak acid and others | sodium acetate-sodium bicarbonate-sodium carbonate, sodium acetate-sodium dihydrogen phosphate-disodium hydrogen phosphate, sodium acetate-disodium hydrogen phosphate-potassium dihydrogen phosphate, sodium lactate-sodium dihydrogen phosphate-sodium carbonate, sodium citrate-sodium bicarbonate-sodium carbonate |
| III | weak acid | acid inorganic salt of polybasic weak acid | sodium bicarbonate-carbonic acid, sodium dihydrogen phosphate-phosphoric acid, |
| | | | disodium hydrogen phosphate-phosphoric acid, disodium hydrogen phosphate-citric acid, sodium bicarbonate-acetic acid, sodium dihydrogen phosphate-phosphoric acid, disodium hydrogen phosphate-citric acid |
| III | weak acid | acid inorganic salt of polybasic weak acid | sodium bicarbonate-carbonic acid, sodium bicarbonate-acetic acid, sodium bicarbonate-citric acid |
| III | weak acid | alkali metal salt of weak organic acid | acetic acid-sodium acetate, citric acid-sodium citrate, citric acid- sodium 2-hydroxypropane-1,2,3-tricarboxylate |
| IV | strong acid | weak acid or others | phthalic acid-hydrochloric acid, citric acid-sodium hydroxide-hydrochloric acid, sodium barbital-hydrochloric acid |

In one embodiment, the acidifying agent or the basifying agent is selected from strong bases, the pH neutralizing agent is one or more selected from one or more of groups consisting of an alkali inorganic salt of polybasic weak acid, a nitrogen-containing weak base, a salt compounded with a strong acid and/or a strong base, an acid inorganic salt of a polybasic weak acid, an alkali metal salt of a weak organic acid, a weak acid, a strong acid, and wherein the concentration of the strong base is ≥0.5%, preferably 0.75-10%; the concentration of the pH neutralizing agent is ≥1%, preferably 2-35%.

In one embodiment, the acidifying agent or the basifying agent is selected from one or more of strong bases, the pH neutralizing agent is selected from one or more of alkali inorganic salts of polybasic weak acid, and wherein the concentration of the strong base is ≥0.75%, preferably 1-10%, more preferably 2-7%; and the concentration of the alkali inorganic salt of polybasic weak acid is ≥2%, preferably 2-20%.

In one embodiment, the acidifying agent or the basifying agent is selected from one or more of strong bases, the pH neutralizing agent is selected from one or more of nitrogen-containing weak bases, and wherein the concentration of the strong base is ≥0.75%, preferably 1-10%, more preferably 2-7%; and the concentration of the nitrogen-containing weak bases is ≥2%, preferably 2-35%.

In one embodiment, the acidifying agent or the basifying agent is selected from one or more of strong bases, the pH neutralizing agent is selected from one or more of acid inorganic salts of polybasic weak acid, and wherein the concentration of the strong bases is ≥0.75%, preferably 1-10%, more preferably 2-7%, and the concentration of the acid inorganic salt of polybasic weak acid is ≥2%, preferably 2-20%.

In one embodiment, the acidifying agent or the basifying agent is selected from one or more of strong bases, the pH neutralizing agent is selected from one or more of alkali metal salts of weak organic acid, and wherein the concentration of the strong base is ≥0.75%, preferably 1-10%, more preferably 2-7%; and the concentration of the alkali metal salt of weak organic acid is ≥2%, preferably 2-35%.

In one embodiment, the acidifying agent or the basifying agent is selected from one or more of strong bases, the pH neutralizing agent is selected from one or more of salts compounded with strong acid and/or strong base, and wherein the concentration of the strong base is ≥0.75%, preferably 1-10%, more preferably 2-5%; the concentration of the salts compounded with strong acid and/or strong base is ≥1%, preferably 1.5-10%.

In one embodiment, the acidifying agent or the basifying agent is selected from one or more of strong bases, the pH neutralizing agent is selected from one or more of groups consisting of one or more of an alkali inorganic salt of polybasic weak acid, a nitrogen-containing weak base, an acid inorganic salt of polybasic weak acid, and wherein the concentration of the strong base is ≥0.75%, preferably 1-10%, more preferably 2-7%; the concentration of the pH neutralizing agent is ≥1%, preferably 2-35%.

In one embodiment, the acidifying agent or the basifying agent is selected from weak bases, the pH neutralizing agent is selected from one or more of groups consisting of one or more of other weak bases, an alkali metal salt of weak organic acid, a weak acid, a strong acid, and wherein the concentration of the weak base is ≥3.0% or ≥5%, or is 2.5-35%, preferably 3.0-35% or 5-35%, and the concentration of the pH neutralizing agent is ≥ 2%, preferably 2-35%, wherein the weak base is selected from an alkali inorganic salt of polybasic weak acid, an acid inorganic salt of polybasic weak acid, or a nitrogen-containing weak base.

In one embodiment, the acidifying agent or the basifying agent is selected from alkali inorganic salts of polybasic weak acid, and wherein the concentration of the alkali inorganic salt of polybasic weak acid is ≥ 2.5%, preferably 2.5-15%.

In one embodiment, the acidifying agent or the basifying agent is selected from nitrogen-containing weak bases, and wherein the concentration of the nitrogen-containing weak base is ≥ 2.5%, preferably 2.5-35%.

In one embodiment, the acidifying agent or the basifying agent is selected from acid inorganic salts of polybasic weak acid, and wherein the concentration of the acid inorganic salt of polybasic weak acid is ≥ 2.5%, preferably 2.5-15%.

In one embodiment, the acidifying agent or the basifying agent is selected from one or more of alkali inorganic salts of polybasic weak acid, the pH neutralizing agent is selected from one or more of groups consisting of one or more of a nitrogen-containing weak base, an acid inorganic salt of polybasic weak acid, an alkali metal salt of weak organic acid, a weak acid, a strong acid, and wherein the concentration of the alkali inorganic salt of polybasic weak acid is ≥2.5%, preferably ≥3.0% or ≥5%, or is 2.5-20%, preferably 3.0-20% or 5-20%, the concentration of the pH neutralizing agent is ≥2%, preferably 2-35%.

In one embodiment, the acidifying agent or the basifying agent is selected from one or more of alkali inorganic salts of polybasic weak acid, the pH neutralizing agent is selected from one or more of acid inorganic salts of polybasic weak acid, and wherein the concentration of the alkali inorganic salt of polybasic weak acid is ≥2.5%, preferably ≥3.0%, or is 2.5-10%, preferably 3.0-10%; the concentration of the acid inorganic salt of polybasic weak acid is ≥2.5%, preferably 2.5-10%.

In one embodiment, the acidifying agent or the basifying agent is selected from one or more of alkali inorganic salts of polybasic weak acid, the pH neutralizing agent is selected from one or more of nitrogen- containing weak bases, and wherein the concentration of the alkali inorganic salt of polybasic weak acid is ≥2.5%, preferably ≥3.0%, or is 2.5-10%, preferably 3.0-10%; the concentration of the nitrogen- containing weak base is ≥2%, preferably 2- 35%.

In one embodiment, the acidifying agent or the basifying agent is selected from one or more of alkali inorganic salts of polybasic weak acid, the pH neutralizing agent is selected from one or more of alkali metal salts of weak organic acid, and wherein the concentration of the alkali inorganic salt of polybasic weak acid is ≥ 2.5%, preferably ≥ 3.0%, or is 2.5-10%, preferably 3.0-10%; the concentration of the alkali metal salt of weak organic acid is ≥ 5%, preferably 5-35%.

In one embodiment, the acidifying agent or the basifying agent is selected from one or more of alkali inorganic salts of polybasic weak acid, the pH neutralizing agent is selected from one or more of weak acids or/and strong acids, and wherein the concentration of the alkali inorganic salt of polybasic weak acid is ≥2.5%, preferably ≥3.0%, or is 2.5-10%, preferably 3.0-10%; the concentration of the weak acid or/and the strong acid is ≥0.5%, preferably 1-15%.

In one embodiment, the acidifying agent or the basifying agent is one or more of acid inorganic salts of polybasic weak acid, the pH neutralizing agent is selected from one or more of groups consisting of one or more of: a nitrogen-containing weak base, an alkali metal salt of weak organic acid, a weak acid, a strong acid, and wherein the concentration of the acid inorganic salt of polybasic weak acid is ≥2.5%, preferably ≥3.0% or ≥5%, or is 2.5-20%, preferably 3.0-20% or 5-20%, the concentration of the pH neutralizing agent is2%, preferably 2-35%.

In one embodiment, the acidifying agent or the basifying agent is selected from one or more of acid inorganic salts of polybasic weak acid, the pH neutralizing agent is selected from one or more of nitrogen-containing weak bases, and wherein the concentration of the alkali inorganic salt of polybasic weak acid is ≥ 2.5%, preferably ≥ 3.0%, or 2.5-10%, preferably 3.0-10%; the concentration of the nitrogen- containing weak base is ≥ 2%, preferably 2- 35%.

In one embodiment, the acidifying agent or the basifying agent is selected from one or more of acid inorganic salts of polybasic weak acid, the pH neutralizing agent is selected from one or more of alkali metal salts of weak organic acid, and wherein the concentration of the alkali inorganic salt of polybasic weak acid is ≥ 2.5%, preferably ≥ 3.0%, or is 2.5-10%, preferably 3.0-10%; the concentration of the alkali metal salt of weak organic acid is ≥ 5%, preferably 5-35%.

In one embodiment, the acidifying agent or the basifying agent is selected from one or more of alkali inorganic salts of polybasic weak acid, the pH neutralizing agent is selected from one or more of weak acids or/and strong acids, and wherein the concentration of the acid inorganic salt of polybasic weak acid is ≥2.5%, preferably ≥3.0%, or is 2.5-10%, preferably 3.0-10%; the concentration of the weak acid or/and the strong acid is ≥0.5%, preferably 1-15%.

In one embodiment, the acidifying agent or the basifying agent is selected from nitrogen-containing weak bases, the pH neutralizing agent is selected from one or more of groups consisting of one or more of: an alkali inorganic salt of polybasic weak acid, an acid inorganic salt of polybasic weak acid, an alkali metal salt of weak organic acid, a weak acid, a strong acid, and wherein the concentration of the nitrogen-containing weak base is ≥2.5%, preferably ≥3.0% or ≥5%, or is 2.5-35%, preferably 3.0-35% or 5-35%, the concentration of the pH neutralizing agent is ≥2%, preferably 2-35%.

In one embodiment, the acidifying agent or the basifying agent is selected fro mone or more of nitrogen-containing weak bases, the pH neutralizing agent is selected from one or more of acid inorganic salts of polybasic weak acid, and wherein the concentration of the nitrogen-containing weak base is ≥2.5%, preferably ≥3.0% or ≥5%, or is 2.5-35%, preferably 3.0-35% or 5-35%; the concentration of the acid inorganic salt of polybasic weak acid is ≥ 2%, preferably 2-10%.

In one embodiment, the acidifying agent or the basifying agent is selected from one or more of nitrogen-containing weak bases, the pH neutralizing agent is selected from one or more of alkali inorganic salts of polybasic weak acid, and wherein the concentration of the nitrogen-containing weak base is ≥2.5%, preferably ≥3.0% or ≥5%, or is 2.5-35%, preferably 3.0-35% or 5-35%; the concentration of the alkali inorganic salt of polybasic weak acid is ≥ 2%, preferably 2-10%.

In one embodiment, the acidifying agent or the basifying agent is selected from one or more of nitrogen-containing weak bases, the pH neutralizing agent is selected from one or more of alkali metal salts of weak organic acid, and wherein the concentration of the nitrogen-containing weak base is ≥2.5%, preferably ≥3.0% or ≥5%, or is 2.5-35%, preferably 3.0-35% or 5-35%; the concentration of the alkali metal salt of weak organic acid is ≥ 2%, preferably 2-35%.

In one embodiment, the acidifying agent or the basifying agent is selected from one or more of nitrogen-containing weak bases, the pH neutralizing agent is selected from one or more of weak acids or/and strong acids, and wherein the concentration of the nitrogen-containing weak base is ≥2.5%, preferably ≥3.0% or ≥5%, or is 2.5-35%, preferably 3.0-35% or 5-35%; the concentration of the weak acid or/and the strong acid is ≥0.5%, preferably 1-15%.

In one embodiment, the acidifying agent or the basifying agent is selected from acids, wherein the acid is selected from one or more of weak acids or strong acids, and wherein the concentration of the strong acid is ≥0.5%, preferably ≥0.75% or ≥1%, or is 0.5-10%, preferably 0.75-10% or 1-10%; or the concentration of the weak acid is ≥2.5%, preferably ≥3.0% or ≥5%, or is 2.5-20%, preferably 3.0-20% or 5-20%.

In one embodiment, the acidifying agent or the basifying agent is selected from weak acids, the pH neutralizing agent is selected from one or more of groups consisting of one or more of a strong base, an alkali inorganic salt of polybasic weak acid, a nitrogen-containing weak base, an acid inorganic salt of polybasic weak acid, an alkali metal salt of weak organic acid, and wherein the concentration of the weak acid is ≥2.5%, preferably ≥3.0% or ≥5%, or is 2.5-20%, preferably 3.0-20% or 5-20%; the concentration of the pH neutralizing agent is ≥1%, preferably 2-35%.

In one embodiment, the acidifying agent or the basifying agent is selected from one or more of weak acids, the pH neutralizing agent is selected from one or more of alkali inorganic salts of polybasic weak acid and/or acid inorganic salts of polybasic weak acid, and wherein the concentration of the weak acid is ≥2.5%, preferably ≥3.0% or ≥5%, or is 2.5-20%, preferably 3.0-20% or 5-20% ; the concentration of the pH neutralizing agent is ≥1%, preferably 2-25%.

In one embodiment, the acidifying agent or the basifying agent is selected from one or more of weak acids, the pH neutralizing agent is selected from one or more of nitrogen-containing weak bases, and wherein the concentration of the weak acid is ≥2.5%, preferably ≥3.0% or ≥5%, or is 2.5-20%, preferably 3.0-20% or 5-20%; and the concentration of the nitrogen-containing weak base is ≥1%, preferably 2-35%.

In one embodiment, the acidifying agent or the basifying agent is selected from one or more of weak acids, the pH neutralizing agent is selected from one or more of alkali metal salts of weak organic acid, and wherein the concentration of the weak acid is ≥2.5%, preferably ≥3.0% or ≥5%, or is 2.5-20%, preferably 3.0-20% or 5-20%; the concentration of the alkali metal salt of weak organic acid is ≥2%, preferably 2-35%.

In one embodiment, the acidifying agent or the basifying agent is selected from strong acids, the pH neutralizing agent is selected from one or more of groups consisting of one or more of: an alkali inorganic salt of polybasic weak acid, a nitrogen-containing weak base, an acid inorganic salt of polybasic weak acid, an alkali metal salt of weak organic acid, a weak acid, and wherein the concentration of the strong acid is ≥0.5%, preferably ≥0.75% or ≥1%, or is 0.5-10%, preferably 0.75-10% or 1-10% ; the concentration of the pH neutralizing agent is ≥1%, preferably 2-35%.

In one embodiment, the acidifying agent or the basifying agent is selected from one or more of strong acids, the pH neutralizing agent is selected from one or more of alkali inorganic salts of polybasic weak acid and/or acid inorganic salts of polybasic weak acid, and wherein the concentration of the strong acid is ≥0.5%, preferably ≥0.75% or ≥1%, or is 0.5-10%, preferably 0.75-10% or 1-10%; the pH neutralizing agent is at a concentration of ≥1%, preferably 2-25%.

In one embodiment, the acidifying agent or the basifying agent is selected from one or more of strong acids, the pH neutralizing agent is selected from one or more of nitrogen-containing weak bases, and wherein the concentration of the strong acid is ≥0.5%, preferably ≥0.75% or ≥1%, or is 0.5-10%, preferably 0.75-10% or 1-10%; the concentration of the nitrogen-containing weak base is ≥1%, preferably 2-35%.

In one embodiment, the acidifying agent or the basifying agent is selected from one or more of strong acids, the pH neutralizing agent is selected from one or more of alkali metal salts of weak organic acid, and wherein the concentration of the strong acid is ≥0.5%, preferably ≥0.75% or ≥1%, or is 0.5-10%, preferably 0.75-10% or 1-10%; the concentration of the alkali metal salt of weak organic acid is ≥1%, preferably 2-35%.

In one embodiment, the acidifying agent or the basifying agent is selected from one or more of strong acids, the pH neutralizing agent is selected from one or more of other weak acids, and wherein the concentration of the strong acid is ≥0.5%, preferably ≥0.75% or ≥1%, or is 0.5-10%, preferably 0.75-10% or 1-10%; the concentration of the other weak acids is ≥1%, preferably 2-25%.

In one embodiment, the total concentration of the acid-base neutralization combination composition is ≥1.5%, preferably 2.25-40%.

In one embodiment, the total concentration of the acid-base neutralization combination composition is also the local administration concentration of the acid-base neutralization combination composition.

In one embodiment, the strong base comprises an alkali metal hydroxide, wherein the alkali metal hydroxide comprises, for example, sodium hydroxide, potassium hydroxide, calcium hydroxide.

In one embodiment, the strong base is sodium hydroxide or/and potassium hydroxide, preferably sodium hydroxide.

In one embodiment, the alkali inorganic salt of polybasic weak acid comprises, for example, sodium phosphate, sodium carbonate, potassium carbonate, borax, preferably sodium carbonate and/or sodium phosphate.

In one embodiment, the acidifying agent or the basifying agent is sodium hydroxide, the pH neutralizing agent is sodium carbonate, and the concentration of the sodium hydroxide is ≥0.5%, preferably 0.75-5%; and the concentration of the sodium carbonate is ≥2%, preferably 2-10%.

In one embodiment, the acidifying agent or the basifying agent is sodium hydroxide, the pH neutralizing agent is sodium phosphate, and the concentration of the sodium hydroxide is ≥0.5%, preferably 0.75-5%; the concentration of the sodium phosphate is ≥2%, preferably 2-10%.

In one embodiment, the acid inorganic salt of polybasic weak acid comprises, for example, sodium dihydrogen phosphate, disodium hydrogen phosphate, sodium bicarbonate, potassium bicarbonate, calcium bicarbonate, sodium bisulfate.

In one embodiment, the acid inorganic salt of polybasic weak acid is sodium bicarbonate and/or disodium hydrogen phosphate.

In one embodiment, the acidifying agent or the basifying agent is sodium hydroxide, the pH neutralizing agent is sodium bicarbonate, and the concentration of sodium hydroxide is ≥0.5%, preferably 0.75-5%; the concentration of sodium bicarbonate is ≥ 2%, preferably 2-10%.

In one embodiment, the acidifying agent or the basifying agent is sodium hydroxide, the pH neutralizing agent is sodium dihydrogen phosphate, and the concentration of sodium hydroxide is ≥0.5%, preferably 0.75-5%; the concentration of the sodium dihydrogen phosphate is ≥2%, preferably 2-10%.

In one embodiment, the acidifying agent or the basifying agent is sodium carbonate, the pH neutralizing agent is sodium bicarbonate, and the concentration of sodium carbonate is ≥1.5%, preferably 3-10%; the concentration of sodium bicarbonate is ≥2.5%, preferably 4-15%.

In one embodiment, the acidifying agent or the basifying agent is disodium hydrogen phosphate, the pH neutralizing agent is sodium dihydrogen phosphate, and the concentration of the disodium hydrogen phosphate is ≥1.5%, preferably 2-7%; the concentration of the sodium dihydrogen phosphate is ≥2%, preferably 3-10%.

In one embodiment, the nitrogen-containing weak base comprises, for example, aqueous ammonia, ammonium chloride, 2-aminoethanol, trometamol, triethanolamine, trihydroxymethylaminomethane, 2-aminoethanol, trometamol, triethanolamine, meglumine, N-ethyl glucamine.

In one embodiment, the alkali metal salt of weak organic acid comprises, for example, potassium hydrogen phthalate, sodium acetate, sodium propionate, sodium butyrate, sodium malonate, sodium lactate, sodium citrate, sodium 2-hydroxypropane-1,2,3-tricarboxylate, sodium malate, sodium dodecyl sulfate.

In one embodiment, the alkali metal salt of a weak organic acid is one or more selected from: sodium acetate, sodium propionate, sodium butyrate, sodium malonate, sodium lactate, sodium citrate, sodium 2-hydroxypropane-1,2,3-tricarboxylate, sodium malate.

In one embodiment, the salt compounded with strong acid or strong base comprises, for example, sodium chloride, potassium chloride, sodium iodide, potassium iodide.

In one embodiment, the acidifying agent or the basifying agent is sodium hydroxide, the pH neutralizing agent is potassium chloride, and wherein the concentration of sodium hydroxide is ≥0.5%, preferably 0.75-5%; and the concentration of the potassium chloride is ≥1%, preferably 1.5-10%.

In one embodiment, the weak acid is selected from one or more of inorganic weak acids or/and organic weak acids, wherein the inorganic weak acids include, for example, phosphoric acid, carbonic acid, boric acid, sulfurous acid ; the organic weak acids include C1-10 aliphatic carboxylic acid substituted with 1-3 hydroxyl groups, for example, acetic acid, hydroxy acetic acid, propionic acid, malonic acid, butyric acid, succinic acid, lactic acid (2-hydroxypropionic acid), citric acid (2-hydroxy-1,2,3-propane-tricarboxylic acid), malic acid (2-hydroxysuccinic acid ) , tartaric acid, oxalic acid, gluconic acid .

In one embodiment, the weak acid is preferably selected from one or more of: carbonic acid, acetic acid, hydroxy acetic acid, propionic acid, malonic acid, butyric acid, succinic acid, lactic acid (2-hydroxypropionic acid), citric acid (2-hydroxy-1,2,3-propane-tricarboxylic acid), malic acid (2-hydroxysuccinic acid), tartaric acid, oxalic acid, gluconic acid; preferably acetic acid.

In one embodiment, the weak acid includes acetic acid, and the concentration of acetic acid is ≥ 3.5%, preferably 5-15%.

In one embodiment, the acidifying agent or the basifying agent is acetic acid, the pH neutralizing agent is sodium bicarbonate, and the concentration of acetic acid is ≥ 5%, preferably 5-15%; the concentration of sodium bicarbonate is ≥2%, preferably 2-10%.

In one embodiment, the acidifying agent or the basifying agent is acetic acid, the pH neutralizing agent is sodium acetate, and the concentration of acetic acid is ≥ 3.5%, preferably 5-15%; the concentration of sodium acetate is ≥ 5%, preferably 5-25%.

In one embodiment, the weak acids include acetic acid and gluconic acid, preferably gluconic acid.

In one embodiment, the strong acids include, for example, hydrochloric acid, sulphuric acid, nitric acid, perchloric acid, selenic acid, hydrobromic acid, hydroiodic acid.

In one embodiment, the strong acid is selected from one or more of: hydrochloric acid, sulphuric acid, nitric acid, preferably hydrochloric acid.

In one embodiment, the acidifying agent or the basifying agent is hydrochloric acid, the pH neutralizing agent is acetic acid, and wherein the concentration of acetic acid is ≥ 3.5%, preferably 5-15%; and the concentration of hydrochloric acid is ≥ 0.5%, preferably 1-5%.

In the scope of the present invention, the local synergist is selected from one or more of a cytotoxic drug and/or a conventional ineffective drug.

In the scope of the present invention, the term "local synergist" refers to a drug that, when administrated by mixing with the described neutralization combination, produces a short-term synergistic effect or/and a medium-term synergistic effect at the administration location.

In one embodiment, a pharmaceutical composition comprising an acidifying agent or a basifying agent, a pH-neutralizing agent, and a local synergist thereof (just take amino acid based nutrients as an example) includes, for example, the compositions listed in Table 2 below.

**Table 2**

| Class | Acidifying agent or basifying agent | pH neutralizing agent | Local synergist | Examples of pharmaceutical compositions |
|---|---|---|---|---|
| V | strong base | weak base or alkali metal salt of weak organic acid | amino acid based nutrients | glycine-sodium carbonate-sodium hydroxide, glycine-sodium acetate-sodium hydroxide, arginine-sodium carbonate-sodium hydroxide, arginine-sodium bicarbonate-sodium hydroxide, arginine-sodium acetate-sodium hydroxide, arginine-sodium citrate-sodium hydroxide, arginine-sodium citrate-sodium hydroxide |
| VI | weak base (alkali inorganic salt of polybasic weak acid) | other weak bases | amino acid based nutrients | arginine-sodium bicarbonate-sodium carbonate, arginine-sodium dihydrogen phosphate-disodium hydrogen phosphate, arginine-sodium dihydrogen phosphate-sodium carbonate, glycine-sodium bicarbonate-sodium carbonate |
| VII | weak acid | alkali inorganic salt of polybasic weak acid | amino acid based nutrients | glycine-sodium carbonate-acetic acid, arginine-sodium carbonate-carbonic acid, glycine-sodium carbonate-phosphoric acid, glycine-arginine- sodium carbonate-acetic acid |
| VII | weak acid | acid inorganic salt of polybasic weak acid | amino acid based nutrients | glycine-disodium hydrogen phosphate-acetic acid, arginine-sodium bicarbonate-carbonic acid, arginine-sodium dihydrogen phosphate-phosphoric acid, glycine-sodium dihydrogen phosphate-phosphoric acid, glycine-sodium bicarbonate-acetic acid |
| VII | weak acid | alkali metal salt of weak organic acid | amino acid based nutrients | glycine-acetic acid-sodium acetate, glycine-citric acid-sodium citrate, glycine-citric acid-sodium citrate |
| VIII | strong acid | acid inorganic salt of polybasic weak acid | amino acid based nutrients | glycine-disodium hydrogen phosphate-hydrochloric acid, arginine-sodium bicarbonate-hydrochloric acid, glycine-sodium dihydrogen phosphate-hydrochloric acid, glycine-sodium bicarbonate-hydrochloric acid |

In the present disclosure, the term "cytotoxic drug" as used herein refers to a drug whose cytotoxicity can be used to effectively treat a local lesion disease (e.g., a solid tumor) by absorption at a safe dose, and is selected from cytotoxic drugs known in the art, preferably cytotoxic drugs, especially anti-tumor chemotherapeutic agents that have been approved or will be approved by the official competent administrative department of China, the United States or Europe (for example, the FDA or China Food and Drug Administration), or have been included or will be included in the official pharmacopoeias of China, the United States or Europe. As used herein, the term "absorption" refers to the pharmacological effect of a drug being absorbed by the blood to form the drug-carrying blood into a target region; and the term "absorbed drug" refers to a therapeutic drug that generates a drug effect mainly through absorption.

In the present disclosure, the term "conventional ineffective drug" as used herein is different from a conventional effective drug (such as an anti-tumor agent), and refers to an agent which may show an effect for specific cells (such as anti-tumor cell effects) in cell experiments, but does not show more effective inhibition than conventional effective compounds in animal experiments through absorption action, and thus has not been approved by a drug regulatory authority (such as the FDA) as a drug for effective treatment of a specific local lesion disease, such as a non-anti-local lesion disease drug, a nutritious agent, an agent for diagnostic, a pharmaceutical excipient, and the like.

In one embodiment, a concentration of the cytotoxic drug is ≥ 50%, preferably 50-100%, of its solubility in the pharmaceutical composition.

In one embodiment, the conventional ineffective drug is ≥ 0.35%, preferably 0.35%-25%.

In one embodiment, the cytotoxic drug is one or more selected from a group consisting of a drug that damages the structure and function of DNA, a drug that is intercalated in DNA and interferes with transcription of RNA, a drug that interferes with DNA synthesis, and a drug that affects protein synthesis; preferably one or more selected from a group consisting of: an alkylating agent, for example cyclophosphamide, and carmustine; a metal platinum complex, for example, cisplatin, and carboplatin; a DNA topoisomerase inhibitor, for example doxorubicin, topotecan, and irinotecan; anti-tumor antibiotics, for example actinomycins, daunorubicin; a pyrimidine antagonist, for example a uracil derivative 5-fluorouracil, ftorafur, tegadifur, cytosine derivative cytarabine, cyclocytidine, 5-azacytidine; taxanes, for example paclitaxel, docetaxel.

In one embodiment, the cytotoxic drug is selected from the alkylating agents (e.g., cyclophosphamide, carmustine, etc.) and wherein the concentration (w/v) of the alkylating agent as antitumor chemotherapeutic agent in the local pharmaceutical composition is 0.5-6%, preferably 0.75-1.5%.

In one embodiment, the cytotoxic drug is selected from the metal platinum complex (e.g., cisplatin, carboplatin, etc.) and wherein the concentration (w/v) of the metal platinum complex in the local pharmaceutical composition is 0.03-0.15%, preferably 0.05-0.15%.

In one embodiment, the cytotoxic drug is selected from the DNA topoisomerase inhibitors (e.g., doxorubicin, topotecan, irinotecan, etc.), and wherein the concentration (w/v) of the DNA topoisomerase inhibitor in the local pharmaceutical composition is 0.05-0.20%, preferably 0.075-0.15%.

In one embodiment, the cytotoxic agent is selected from the antitumor antibiotics (e.g., actinomycins, daunorubicin, etc.) and wherein the concentration (w/v) of the antitumor antibiotics in the local pharmaceutical composition is 1-4%, preferably 1-2%.

In one embodiment, the cytotoxic drug is selected from pyrimidine antagonists (e.g. a uracil derivative 5-fluorouracil, ftorafur, tegadifur, cytosine derivative cytarabine, cyclocytidine, 5-azacytidine, etc.), and wherein the concentration (w/v) of pyrimidine antagonists in the local pharmaceutical composition is 0.5-2%, preferably 0.75-1.5%.

In one embodiment, the cytotoxic drug is selected from the taxanes (e.g., paclitaxel, docetaxel, etc.) and wherein the concentration (w/v) of the taxanes in the local pharmaceutical composition is 0.5-2%, preferably 0.75-1.5%.

In one embodiment, the conventional ineffective drug is selected from one or more of groups consisting of one or more of: amino acid based nutrients, carbohydrate nutrients, lipid nutrients, pigment and aromatic compounds, salicylic acid compounds, quinine compounds, blood volume expanders, probiotic components. Preferably, the blood volume expanders comprise polymer blood volume expanders.

In the scope of the present invention, the term "amino acid based nutrients" refers to amino acids and their small molecule (molecular weight <3000) derivatives with nutritional and healthcare effects.

In the scope of the present invention, the term "carbohydrate nutrients" as used herein refers to a carbohydrate compound with nutritional and healthcare effects, preferably selected from a monosaccharide, a sugar polymer and a sugar derivative having nutritional and health effects, more preferably selected from carbohydrate nutritious drugs and carbohydrate excipients with nutritional and healthcare effects included in official pharmacopoeias or guidelines of China, the United States or Europe, which comprises, for example, monosaccharides or their derivatives from a group consisting of: glucose, ribose, deoxyribose, xylose, fructose, galactose, fucose.

In the scope of the present invention, the term "lipid nutrients" is used to refer to a lipid compound with nutritional and healthcare effects, preferably a lipid compound with nutritional and healthcare effects included in the official pharmacopoeias or guidelines of China, the United States or Europe, and more preferably is one or more selected from a fat, a fatty acid, a fat emulsion and an adipoid.

In the scope of the present invention, the term "pigment and aromatic compound" refers to a pharmaceutically acceptable aromatic compound that makes a target region selectively absorb or reflects light of a particular wavelength, which can comprise, for example, vital dyes, photosensitizers, and colored chemotherapeutic drugs. The vital dyes can comprise, for example, one or more of the following organic dyes and their derivatives: methylene blue (including its hydrate), patent blue, isosulfan blue, Toluidine Blue, Trypan Blue, Basic Blue, Eosin, Basic Fuchsin, Crystal Violet, Gentian Violet, Neutral Red, Janus Green B, Safranin, Bengal Red, etc. Photosensitizers can comprise, for example, one or more of the following: mixed porphyrin photosensitizers, porphyrins (e.g., porphyrin, porphin, purpurin, endogenous porphyrin) and their derivatives, phthalocyanine compounds, bacteriochlorin compounds, condensed cyclic quinone compounds, benzoporphyrin derivatives, 5-aminolevulinic acid, dihydroporphin compounds, etc. Colored chemotherapeutic drugs can comprise, for example, one or more of the following: nitrophenolic compounds, flavonoids (e.g., anthocyanins, genisteins, etc.), isohexenyl naphthoquinone compounds (e.g., shikonins), etc. Take methylene blue as an example, its derivatives are usually also dyes, such as 1, 9-dimethyl methylene blue, 1-methyl methylene blue, etc. Some pigment and aromatic compounds, such as methylene blue, are both vital dyes, photosensitizers, and colored chemotherapeutic drugs.

In the scope of the present disclosure, the term "salicylic acid compound" as used herein refers to a salicylic acid and a derivative thereof. The chemical name of salicylic acid is 2-hydroxybenzoic acid. The salicylic acid derivative may be any suitable one known to a person skilled in the art, which may include, for example, a salicylic acid derivative containing or not containing a metal compound. The former may include, for example, sodium salicylate, magnesium salicylate, zinc salicylate, metal element complexes (such as copper aspirin), and the like, while the latter may include, for example, acetylsalicylic acid (Aspirin), Aspisol, difluorobenzenesalicylic acid, aminosalicylic acid, p-aminosalicylic acid, N-phenylanthranilic acid, salicylanilide, o-ethoxybenzamide, phenyl salicylate, methyl salicylate, methyl p-hydroxybenzoate, ethyl p-hydroxybenzoate, di-salicylate, dicoumarin, and their pharmaceutically acceptable derivatives.

In the scope of the present disclosure, the term "quinine compounds" as used herein refers to a pharmaceutically acceptable quinine and a structural analog thereof, such as quinine and an isomer and a pharmaceutically acceptable salt thereof. For quinine, its isomer may be, for example, quinidine, cinchonine, and cinchonidine, and its salts may be, for example, quinine hydrochloride, quinine dihydrochloride, quinine sulfate, and the like.

In one embodiment, the concentration (w/v) of the amino acid-based nutrient is ≥ 5%, preferably 10-35%.

In one embodiment, the concentration (w/v) of the carbohydrate nutrients is ≥ 5%, preferably 5-40%.

In one embodiment, the concentration of the lipid nutrients is greater than 5%, preferably 10-30%.

In one embodiment, the concentration (w/v) of the pigment and aromatic compound is ≥ 0.35%, preferably 0.35-10%.

In one embodiment, the concentration (w/v) of the salicylic acid compounds is ≥ 2%, preferably 2-30%.

In one embodiment, the concentration (w/v) of the quinine compounds is ≥ 2%, preferably 2-10%.

In one embodiment, the amino acid based nutrient is selected from one or more of basic amino acid based nutrients and/or non-basic amino acid based nutrients, wherein the basic amino acid based nutrient is, for example, arginine, lysine, histidine, preferably arginine; the non-basic amino acid based nutrient is, for example, one or more of a group consisting of neutral amino acids, acidic amino acids, amino acid salts, wherein the neutral amino acid is, for example, glycine, tryptophan, tyrosine, serine, cysteine, methionine, asparagine, glutamine, threonine, alanine, valine, leucine, isoleucine, phenylalanine, proline; the acidic amino acid is, for example, aspartic acid, glutamic acid; the amino acid salts comprises salts formed by an amino acid as described above with an acid, for example lysine hydrochloride, histidine hydrochloride, glutamic acid hydrochloride, cysteine hydrochloride, arginine hydrochloride, glycine sulfate, iron glycine sulfate, lysine hydrochloride, aspartic hydrochloride.

In one embodiment, the amino acid based nutrient is preferably one or more amino acids and their derived salts selected from: arginine, lysine, glycine, tryptophan, serine, cysteine, glutamine, proline.

In one embodiment, the concentration of the amino acid based nutrient is preferably ≥ 5%, preferably 7.5-35%.

In one embodiment, the amino acid based nutrient comprises arginine.

In one embodiment, the amino acid based nutrient is arginine, and wherein the concentration of arginine is ≥ 10%, preferably 10-20%.

In one embodiment, the acidifying agent or the basifying agent is sodium hydroxide, the pH neutralizing agent is sodium bicarbonate, the amino acid based nutrient is arginine, and wherein the concentration of sodium hydroxide is ≥ 0.5%, preferably 0.75-5%; the concentration of sodium bicarbonate is ≥ 2.5%, preferably 4-15%; and the concentration of arginine is ≥ 10%, preferably 10-20%.

In one embodiment, the acidifying agent or the basifying agent is sodium carbonate, the pH neutralizing agent is sodium bicarbonate, the amino acid based nutrient is arginine, and wherein the concentration of sodium carbonate is ≥1.5%, preferably 2-10%; the concentration of sodium bicarbonate is ≥1%, preferably 1-5%; and the concentration of arginine is ≥10%, preferably 10-20%.

In one embodiment, the amino acid-based nutrient comprises glycine.

In one embodiment, the amino acid based nutrient is glycine and wherein the concentration of glycine is ≥ 10%, preferably 10-25%.

In one embodiment, the amino acid based nutrient comprises lysine hydrochloride.

In one embodiment, the amino acid based nutrient is a mixture of 2 or more amino acid based nutrients.

In one embodiment, the carbohydrate nutrient is selected from one or more of: glucose, fructose, oligochitosan, glucosamine, lactulose, sorbitol, ribose, sorbose, mannose, galactose, sucrose, lactose, trehalose, xylo-oligosaccharide, fructo-oligosaccharide, mannose oligosaccharide, xylitol; and more preferably is selected from one or more of: glucose, sodium gluconate, oligochitosan, glucosamine, lactulose, ribose, oligomannose, xylitol; and the concentration (w/v) of the carbohydrate nutrient in the pharmaceutical composition is ≥10%, preferably 10-40%.

In one embodiment, the lipid nutrient is selected from one or more of a vegetable oil, eicosapentaenoic acid (EPA), docosahexaenoic acid (DHA), long-chain fat emulsion, medium-chain fat emulsion, phospholipids, and the concentration (w/v) of the lipid nutrient in the pharmaceutical composition is ≥ 4%, preferably 4-25%.

In one embodiment, the pigment and aromatic compounds is selected from one or more of methylene blue, patent blue, isosulfan blue, Bengal red, and in the pharmaceutical composition, the concentration (w/v) of the pigment and aromatic compounds is ≥ 0.35%, preferably 0.5-10%.

In one embodiment, the salicylic acid compounds is selected from one or more of salicylic acid, acetylsalicylic acid, Aspisol, and in the local pharmaceutical composition, the concentration (w/v) of the salicylic acid compounds is ≥ 5%, preferably 5-10%.

In one embodiment, the quinine compounds is selected from one or more of: quinine hydrochloride, quinine dihydrochloride and quinine sulfate, and in the local pharmaceutical composition, the concentration (w/v) of the quinine compounds is ≥ 3%, preferably 3-6%.

In one embodiment, the blood volume expander comprises at least one of a dextran blood volume expander, a starch derivative blood volume expander, a gelatin derivative blood volume expander, a synthetic blood volume expander. In one embodiment, the dextran blood volume expander is selected from at least one of dextran 10, dextran 40, dextran 70; the starch derivative blood volume expander is selected from at least one of carboxyethyl starch 20, carboxyethyl starch 40, carboxyethyl starch 130/0.38-0.45, carboxyethyl starch 200/0.5. The gelatin derivative blood volume expander can be oxidized polygelatin and/or succinyl gelatin; the synthetic blood volume expander can be fluorocarbon man-made blood and/or polyvinylpyrrolidone. In one embodiment, the pharmacological concentration of the expander can be 2-30%, preferably 2-25% or 2-20%.

In one embodiment, the probiotic component comprises at least one of inactivated probiotics, a probiotic water-soluble component, a probiotic water-insoluble component particle, a probiotic semi-fluidic component. The probiotic component is selected from one or more of a probiotic water-soluble component, a probiotic semi-fluidic component, a probiotic component water-insoluble particle, and inactivated probiotics, and has low bacterial immunogenicity. As an example of the present invention, the probiotic component is selected from a probiotic water-soluble component and/or engineered analogues thereof (e.g. a water-soluble derivative of a probiotic polysaccharide), and the composition is a solution composition. In one embodiment, the probiotic water-soluble component is selected from a group comprising one or more of the following and derivatives thereof: a disrupted probiotic supernatant component, a probiotic extract, a probiotic intracellular water-soluble component, a probiotic polysaccharide, a probiotic nucleic acid, preferably is selected from one or more of the following water-soluble pharmaceutical groups: a probiotic polysaccharide, a probiotic polysaccharide derivative, a probiotic nucleic acid. In one embodiment, the concentration of the probiotic component is 0.25-25%, 0.25-20%, preferably 0.5-15%, more preferably 1-15% or 5-15%.

In one embodiment, the probiotic is selected from one or more of the group comprising natural or/and engineered bacteria: probiotic Bacillus, probiotic Lactobacillus, probiotic Bifidobacterium, probiotic Fungus. In one embodiment, the Bacillus comprises one or more selected from a group comprising of: Bacillus licheniformis, Bacillus subtilis, Bacillus pumilus, Bacillus natto; the Lactobacillus comprises one or more selected from a group comprising of Lactobacillus acidophilus, Lactobacillus casei, Lactobacillus plantarum, Lactobacillus pumilus and Lactobacillus fermentum; the Bifidobacterium comprises one or more selected from a group comprising of Bifidobacterium longum, Bifidobacterium adolescentis, Bifidobacterium breve, Enterococcus faecium, and Streptococcus fecalis; said Fungus comprises one or more selected from a group comprising of yeasts and Brettanomyces bruxellensis (Saccharomyces boulardii), wherein said yeasts comprises one or more selected from a group comprising of Saccharomyces cerevisiae, Saccharomyces delbouillis, Saccharomyces Wickham, Saccharomyces Pichia, Candida utilis, Whey yeast. In one embodiment, the probiotics comprise selected from Saccharomyces cerevisiae or/and Brettanomyces bruxellensis(Saccharomyces brucella).

The pharmaceutical composition according to the present disclosure may be in any dosage form suitable for local administration which comprises an active ingredient (the pharmaceutical composition, and optionally the other drugs as described above), preferably the dosage form is an injection (preferably local injection), a liquid for external use, an atomizing preparation, and the like.

In the context of the present invention, the term "injection" as used herein refers to a sterile preparation for in vivo administration which comprises an active ingredient and a liquid carrier. The injections are divided according to the administration mode into local injections, intravenous injections, and the like, and the intravenous injections may be used as local injections only after reaching a given local administration concentration. Injections are divided according to types of commodities into liquid injections, injectable powder preparations, and the like. Injectable powder preparations contain sterile dry powder and a solvent, where the sterile dry powder contains part or all of the active ingredients, and the solvent contains all liquid carriers. The concentration of the active ingredient in the injection is the concentration of the active ingredient in the mixture of the active ingredient with all the liquid carriers, and is usually the concentration of the active ingredient in the liquid medicine at the end point (such as a needle hole, a catheter outlet, and the like) of a local administration device (a syringe, a puncture outfit, an infusion catheter, and the like). For injectable powder preparations, the concentration of the active ingredient is the concentration of the active ingredient in the mixture of sterile dry powder and a solvent (for example, a reconstituted solution, or the pharmaceutically acceptable liquid carrier). In the context of the present invention, the term "liquid for external use" refers to a liquid medicine that contains an active ingredient and a liquid carrier, and is administered to a body surface [such as skin, mucous membrane (such as ocular mucosa, nasal mucosa, and the like) or/and a cavity (such as oral cavity, rectum, vagina, urethra, nasal cavity, ear canal, and the like)], and includes, for example, lotion, liniment, drop, gargle, paint and the like. For local administration, the liquid medicine is generally provided by a local administration device such as a lotion bottle, a drip bottle, a dropping pipette, a gargle bottle, a cotton swab and the like. The concentration of the active ingredient in the liquid for external use is the concentration of the active ingredient in the liquid medicine.

In the scope of the present invention, the term "atomizing preparation" refers to a dosage form that contains an active ingredient and a liquid carrier and that can be used to spray the above liquid medicine through pressure when being used. The atomizing preparation may be administered to a skin and a mucous membrane (such as eye mucosa, nasal mucosa, and the like) or/and a cavity (such as oral cavity, rectum, vagina, urethra, nasal cavity, ear canal, and the like), and includes, for example, an aerosols, a sprays, a nebula and the like. In local administration, atomization of a liquid medicine is often formed by a local administration device such as an atomizer, a nebulizer, and a sprayer. After the medicine is sprayed to a target position, it accumulates to form a liquid medicine, the composition of which is approximately the same as that of the liquid medicine before atomization. As such, the concentration of the active ingredient in the atomizing preparation can be represented by the concentration of the active ingredient in the liquid medicine before atomization.

A person skilled in the art would understand that, according to the technical solutions of the present invention, the composition of the present invention should be prepared into a dosage form that can be locally administered to a target area, preferably a local pharmaceutical dosage form.

According to the preparation method of the present invention, the preparation of the pharmaceutical composition of the present invention includes the following steps: preparing a liquid drug comprising the essential component(s) (for example, the pharmaceutical composition), and optionally other substances. The liquid drug may be a solution (for example, a solution in a hydrophilic solvent, preferably an aqueous solution), a suspension, or an emulsion. When the liquid pharmaceutical composition is a suspension, the dispersion medium therein can be any suitable one known to a person skilled in the art, such as a micro-material or a nano-material. When the liquid pharmaceutical composition is an emulsion, the dispersion medium therein may be any suitable one known to a person skilled in the art, such as vegetable oil, synthetic oil or semi-synthetic oil that can be used for injection. The vegetable oil may be, for example, cottonseed oil, almond oil, olive oil, castor oil, sesame oil, soybean oil, and peanut oil.

According to the preparation method of the present invention, the concentrations of the pharmaceutical composition and other drugs are greater than or equal to their concentrations in the pharmaceutical composition of the present invention. When such concentration is greater than the concentration in the pharmaceutical composition of the present invention, it can be further diluted for use.

According to an embodiment of the present disclosure, the liquid injection of the pharmaceutical composition of the present invention can be prepared by a method including the following steps: 1) adding the essential components (for example, the pharmaceutical composition), optionally other components in a required amount based on a local administration concentration to water, to prepare a liquid; 2) adding other optional drugs in a required amount based on the local administration concentration to the liquid prepared in 1), mixing them to uniform, thus obtaining a liquid drug; and 3) sterilizing the liquid drug prepared in 2) and making into a liquid injection. When used, the sterilized liquid drug in the form of the liquid injection formulation can be used directly or after diluted, as a liquid medicine for local administration.

According to an embodiment of the present disclosure, the injectable powder of the pharmaceutical composition of the present invention can be prepared by a method including the following steps: preparing a sterile dry powder of the pharmaceutical composition in a required amount based on a local administration concentration; and preparing a sterile vehicle containing other optional components in a required amount of based on the local administration concentration. The sterile dry powder is preferably a sterile lyophilized dry powder, and a preparation method of the sterile dry powder includes: 1) preparing a solution containing an amino acid based nutrient, water soluble weak neutralizing agent, and optionally other components; 2) sterilizing, filtering and packaging; 3) lyophilizing; 4) and plugging and capping. Process conditions for lyophilizing include, for example, a pre-freezing condition in which a pre-freezing temperature -45°C is kept for 4 hours; a sublimation drying condition in which the temperature is raised to -15°C at a rate of 0.1°C/min and kept at least for 10 hours; and an adsorption drying condition in which a temperature of 30°C is kept for 6 hours. In use, the sterile dry powder of the injectable powder preparation is reconstituted in a sterile solvent to form a reconstituted drug, which can be used directly or diluted to be used as a liquid drug for local administration.

According to an embodiment of the preparation method of the present invention, the liquid for external use of the pharmaceutical composition of the present invention is prepared by a method comprising the following steps: adding the pharmaceutical composition and optionally other ingredients in a required amount based on a local administration concentration to a solvent to prepare a liquid drug. In use, the liquid drug can be used directly or diluted to be used as the liquid for external use for local administration.

According to an embodiment of the preparation method of the present invention, the atomizing preparation of the pharmaceutical composition of the present invention can be prepared by a method including the following steps: 1) adding the pharmaceutical composition and an atomizing excipient in a required amount based on a local administration concentration to a solvent to prepare a liquid; 2) adding other optional ingredients (such as a water-soluble acid) in a required amount based on the local administration concentration to the liquid prepared in 1), and mixing them evenly to obtain a liquid drug. atomizing excipients commonly used include, for example, glycerin, polysorbate-80, benzalkonium chloride, microcrystalline cellulose-sodium carboxymethyl cellulose and the like. In use, the liquid drug is added to an atomizer (such as a sprayer) and locally administered to a target area in a form of mist under an atomization action, where these mists are accumulated into the liquid drug at the target area.

According to the principles of the above methods, a person skilled in the art can adopt any suitable specific method to prepare a variety of specific dosage forms containing the composition of the present invention. For example, variants made to the pharmaceutical composition of the present invention may include: containing different types and concentrations of the pharmaceutical composition, containing different types and concentrations of other drugs, containing different types and concentrations of other additives (such as an analgesic, an activator, and the like).

In the present disclosure, the pharmaceutical composition is mainly used to prevent and treat a local lesion disease, especially a refractory local lesion disease through local administration.

In the context of the present invention, the term "local lesion disease" refers to a disease showing a local lesion symptom, and the term "local lesion" refers to an original or secondary abnormality in a structure, morphology or function of a local body part of an animal (preferably human), which may include, for example, one or more of the followings: a tumor body, non-neoplastic enlargement, local inflammation, abnormal secretory function of secretory gland, and the like. The local body part may be any suitable one known to a person skilled in the art. For example, it may be a local body part that includes one or more of the following organs: a secretory organ in which the secretory system is located, a cardiovascular organ in which the blood circulation system is located, skin, and the like.

Local administration requires that the pharmaceutical composition (a local active ingredient, ratio of the constitution and concentration of the ingredient(s)) can be administered to a tissue in which the local lesion is located by an interventional means, and generates a desired therapeutic effect in the tissue. For example, when the lesion is a tumor, the local tissue is the tumor body in which the tumor cells are located. When the lesion is non-neoplastic enlargement, the local tissue is an abnormality such as a swollen mass, such as hyperplasia, cyst, nodules, and the like. When the lesion is a local inflammation, the local tissue is an inflamed area, such as an inflamed swollen body. When the lesion is abnormal secretion, the local tissue is a source of the abnormality or a secretory gland in which such an abnormal secretion is located. For another example, when the disease is abnormal insulin secretion, the source of the abnormality is the pancreatic islet, and the local tissue is the pancreatic islet or the pancreas in which the pancreatic islet is located. When the disease is a skin disease, the local tissue is the lesion skin or an accessory organ of the lesion skin.

Specifically, in the present disclosure, the local lesion includes a tumor, non-neoplastic enlargement, local inflammation, abnormal secretory gland function, and a skin disease.

Within the context of the present invention, the term "tumor" refers to a swollen mass formed due to abnormal proliferation of cells or mutated cells, and includes a solid tumor. The term "solid tumor" refers to a tumor with a tumor body, and can be a tumor formed due to any pathology (malignant and non-malignant) and at any stage, including, for example, the following group classified according to tumor cell types: epithelial cell tumors, sarcomas, lymphoma, germ cell tumor, blastoma; and tumors named after an organ or tissue in which the tumor cell concentration area is located, including, for example, tumors named after the following organs or tissues: skin, bone, muscle, breast, kidney, liver, lung, gallbladder, pancreas, brain, esophagus, bladder, large intestine, small intestine, spleen, stomach, prostate, testis, ovary or uterus.

Specifically, the malignant tumor includes, for example, breast cancer, pancreatic cancer, thyroid cancer, nasopharyngeal cancer, prostate cancer, liver cancer, lung cancer, intestinal cancer, oral cancer, esophageal cancer, stomach cancer, laryngeal cancer, testicular cancer, vaginal cancer, uterine cancer, ovarian cancer, and the like.

The non-malignant tumor includes, for example, breast tumor, pancreatic tumor, thyroid tumor, prostate tumor, liver tumor, lung tumor, intestinal tumor, oral tumor, esophageal tumor, stomach tumor, nasopharyngeal tumor, laryngeal tumor, testicular tumor, vaginal tumor, uterine tumor, fallopian tube tumor, ovarian tumor, and the like.

In an embodiment, the local lesion disease includes non-neoplastic enlargement. The term "non-neoplastic enlargement" refers to enlargement other than tumors, including, for example, hyperplasia (such hyperplasia of breast, pancreas, thyroid, parathyroid, prostate, and the like), cysts (such as cysts of breast, thyroid, parathyroid, and the like), nodules (such as nodules in breast, thyroid, parathyroid, and the like), abnormal venous mass (such as hemorrhoids), local inflammation and swelling, microbial infection and swelling, and the like. The hemorrhoids include internal hemorrhoids, external hemorrhoids, and mixed hemorrhoids.

In an embodiment, the local lesion disease includes local inflammation, especially refractory inflammation. Within the context of the present invention, the term "local inflammation" refers to non-neoplastic inflammation of a local body part, including, for example, alterative inflammation, exudative inflammation, and proliferative inflammation, which may be any suitable one known to a person skilled in the art, for example, including one or more of the followings: arthritis, mastitis, pancreatitis, thyroiditis, prostatitis, hepatitis, pneumonia, enteritis, stomatitis, pharyngitis, periodontitis, esophagitis, gastritis, gastric ulcer, rhinitis, sinusitis, laryngitis, tracheitis, bronchitis, vaginitis, metritis, salpingitis, oophoritis, and the like.

In one embodiment, the local lesion disease includes a skin disease, especially a refractory skin disease. Within the context of the present invention, the term "skin disease" refers to a lesion that is primary or secondary to a skin or a skin accessory organ, which may be any suitable one known to a person skilled in the art, for example, including one or more of the followings: skin cancer, non-malignant skin tumors, viral skin diseases (such as herpes, warts, rubella, hand-foot-and-mouth disease), bacterial skin diseases (such as impetigo, boils, leprosy), fungal skin diseases (such as various ringworms), sexually transmitted diseases (such as syphilis, gonorrhea, and condyloma acuminatum), allergic and autoimmune skin diseases (such as contact dermatitis, eczema, urticaria), physical skin diseases (such as solar skin diseases, frostbite, corns, cracked skin of hand and foot, pressure sores), connective tissue diseases (such as lupus erythematosus), pigmented skin diseases (such as freckles, pigmented moles, various spots), skin appendage diseases (such as acne, rosacea, seborrheic dermatitis, alopecia areata, baldness, hyperhidrosis and bromhidrosis).

In an embodiment, the local lesion disease includes abnormal secretory function of secretory gland. Within the context of the present invention, the term "secretory gland" refers to a structure that is formed by gland cells or gland cell population and performs a secretion function, including exocrine glands and endocrine glands. The abnormal secretory function of secretory gland includes hyperfunction of secretory glands (such as hyperthyroidism) and hypofunction of secretory glands (such as hypothyroidism, hypoinsulinism (a type of diabetes)).

In an embodiment, the local lesion disease includes a cardiovascular disease. Interventional therapy has become an important treatment for a cardiovascular disease. The cardiovascular disease includes, for example, hemangioma, hypertrophic obstructive cardiomyopathy, atrial fibrillation, arrhythmia, arterial embolism and the like.

The local pharmaceutical composition of the present invention is a therapeutic drug. When used to prevent and treat a local lesion disease, the pharmaceutical composition can also be applied in combination with other interventional therapies, systemic chemotherapy, immunotherapy, photodynamic therapy, sonodynamic therapy, surgical intervention or a combination thereof, to further increase the efficacy.

In the present disclosure, the pharmaceutical composition is mainly used to prevent and treat a local lesion disease through local administration.

In the use and method for local treatment and prevention of a local lesion disease according to the present application, the comprised acidifying agent or basifying agent, pH neutralizing agent, and their local synergist thereof are locally administered at their concentrations or amount ratios in the local pharmaceutical composition. This administration at the concentrations or amount ratios can provide a synergistic effect of local response.

Based on the studies described in more detail below, although specific mechanism has yet to be further studied, the pharmaceutical composition of the present invention has been shown to be effective in promoting damage in a relevant structure of a tissue where a local lesion is located (such as a lesion tissue) while minimizing damage to the patient's normal tissues, so as to achieve a safe and effective pharmaceutical effect of treating a local lesion disease.

### Examples

The following specific examples are used to further illustrate the present invention, but not as a limitation to the present invention. In the following examples, all experimental animals were conducted in accordance with relevant regulations and industrial self-discipline. Unless otherwise specified, all tests were carried out according to conventional methods.

The materials and reagents used in the following specific examples can be obtained from commercial sources unless otherwise specified. Some basifying agent, water-soluble electrolytes and antitumor drugs used in the following Examples are listed in the following table.

**Table 3**

| Class | Compound | CAS No. | Class | Compound | CAS No. |
|---|---|---|---|---|---|
| 1 | sodium hydroxide | 1310-73-2 | 7 | phosphoric acid | 7664-38-2 |
| | potassium hydroxide | 1310-58-3 | | carbonic acid | 463-79-6 |
| | calcium hydroxide | 1305-62-0 | | boric acid | 10043-35-3 |
| 2 | hydrochloric acid | 7647-01-0 | | acetic acid | 64-19-7 |
| | sulphuric acid | 7664-93-9 | | gluconic acid | 147-85-3 |
| | nitric acid | 7697-37-2 | | lactic acid (2-hydroxypropanoic acid) | 50-21-5 |
| 3 | disodium hydrogen phosphate | 10039-32-4 | | citric acid | 52-89-1 |
| | sodium carbonate | 497-19-8 | 8 | arginine | 74-79-3 |
| | potassium carbonate | 584-08-7 | | lysine | 56-87-1 |
| 4 | 2-aminoethanol | 141-43-5 | | glycine | 56-40-6 |
| | trometamol | 77-86-1 | | tryptophan | 153-94-6 |
| | triethanolamine | 102-71-6 | | cysteine | 52-90-4 |
| 5 | sodium acetate | 127-09-3 | 9 | sodium chloride | 7647-14-5 |
| | sodium lactate | 312-85-6/ 72-17-3 | | potassium chloride | 7447-40-7 |
| | sodium citrate | 6132-04-3 | 10 | 5-fluorouracil | 51-21-8 |
| | Sodium citrate | | | glucose | 50-99-7 |
| 6 | sodium dihydrogen phosphate | 7558-80-7 | | docosahexaenoic acid (DHA)) | 6217-54-5 |
| | sodium bicarbonate | 144-55-8 | | methylene blue | 7220-79-3 |
| | potassium bicarbonate | 298-14-6 | | quinine dihydrochloride | 60-93-5 |

| | | | | | |
|---|---|---|---|---|---|
| Class : Class 1 is strong bases; Class 2 is strong acids; Class 3 is alkali inorganic salts of polybasic weak acid; Class 4 is other weak bases; Class 5 is alkali metal salts of organic weak acid; Class 6 is acid inorganic salts of polybasic weak acid; Class 7 is weak acids; Class 8 is amino acid compounds; Class 9 is salts compounded with strong acid and strong base; Class 10 is other drugs. | | | | | |

The experimental animals used in the following examples are SPF (Specific Pathogen Free) grade animals purchased from professional laboratory animal companies. For example, there are two types of mice: BALB/c mice and nude mice, wherein the nude mice are mutant line (BALB/c-nu) mice obtained by introducing a nude gene (nu) into BALB/c mice. The mice are healthy females of 6-8 week old, weighing 17.5-20.5 g.

Animal modeling of subcutaneous transplantation of cells to generate an animal with local lesion (e.g., tumor body) is prior art. The modeling is successful when the tumor body grows to a desired volume (e.g., 50-500 mm³ in tumor-bearing mice). After modeling, the animals were divided into several groups, with 6 animals per group and observed periodically to determine general status, body weight, food intake, animal graft-versus-host disease, tumor volume, tumor weight, survival time, etc. Preferably, the local lesion models comprise a tumor body comprising tumor cells (representing the tumor body), fibroblast-containing nodules (representing a fibroblast-comprising tumor body and other local lesions), and normal biopsies (representing local lesions comprising normal tissues), respectively.

Local lesion (e.g., tumor) volume (V), relative local lesion (e.g., tumor) proliferation rate (R), local lesion (e.g., tumor body) inhibition rate (r'), and tumor inhibition rate (r) were calculated according to the following equation:
Volume of the local lesion (e.g. tumor body): V=0.5×a×b², wherein a and b represent the length and width of the local lesion, respectively;
Relative local lesion (e.g., tumor body) proliferation rate R = TRTV/CRTV ×100, wherein TRTV and CRTV are the local lesion volumes in the study group and negative control group, respectively;
Relative local lesion (e.g., tumor body) volume = Vₜ/V₀, where V₀, Vt represent the tumor volumes on the first day of drug administration and day t of drug administration after grouping, respectively;
Local lesion (e.g., tumor body) inhibition rate (r') = 100% - R, wherein R is the tumor proliferation rate.
Tumor inhibition rate r=(CW-TW)/CW×100%, wherein TW and CW are the mean tumor weights of the study group and negative control group, respectively.

In the following examples, the pharmaceutical efficacy of drug i is designated as Ei, which could be represented as ri' or ri. The action type of a drug, i.e. pharmacology, could be studied by the pharmaceutical efficacy, in particular the pharmaceutical efficacy of the same drug in different regimens. For example, when the difference in pharmaceutical efficacy of drug i between regimens X and Y is not significant (e.g., Ei _{X}/Ei _{Y}<200%), it indicates that the pharmacology of the drug is same; when the difference in pharmaceutical efficacies of drug i between regimens X and Y is significant (e.g., Ei_{X}/Ei_{Y}>200%), it indicates that the pharmacology of the drug i in regimen X greatly exceeds the expected range of its pharmacology in regimen Y in terms of dynamics and is likely to involve a new pharmacology different from that of regimen Y. If two drugs show significantly different Ei_{X}/Ei_{Y} relationships, they are likely to involve different pharmacology; if two drugs show similar Ei_{X}/Ei_{Y} relationships, they are likely to involve the same pharmacology, or at least involve similar pharmacology, such as chemical ablation-like and chemical ablation pharmacology.

In the following examples, experimental results (e.g., tumor weight, tumor volume, lesion tissue volume) were expressed as mean ± standard deviation (x ± s), and the differences between two experimental animal groups and group mean value were tested for significance using the statistical software SPSS13.0 or SPSS19.0, and the test was performed using the statistical quantity t. The test level of α=0.05, P<0.05 indicated a statistically significant difference, while P > 0.05 was not statistically significant.

Positive controls for chemotherapy comprised a classical cytotoxic drug (e.g., 0.5-1% 5-fluorouracil, which had a tumor inhibition rate of ≥ 30% under the conditions of the following examples) and a classical chemical ablation agent (e.g., 75-99% ethanol, which had a tumor inhibition rate of ≥15% under the conditions of the following examples). Immune-enhancing positive controls comprised immune-enhancing agents such as cytokines (e.g., interleukin-12), bacterial components (e.g., attenuated bacterial vaccines, etc.), and vaccine adjuvants, etc..

Within the scope of the present invention, the combination of drug A and drug B was designated as B/A, and their co-administration effect was determined by q = actual co-administration effect/theoretically expected purely additive effect, wherein the pharmaceutical efficacies of drug A alone and drug B alone was designated as E_{A} and E_{B}, respectively, and the actual co-administration pharmaceutical efficacies of A/B was designated as E_{A+B}, such as for the tumor inhibition rate. When q = 1, the actual co-administration effect was in accordance with the theoretical expectation, showing an additive effect; when q < 1, the actual co-administration effect was weaker than the theoretical expectation, showing an antagonistic effect; when q > 1, the actual co-administration effect exceeded the theoretical expectation, showing a synergy.

The method for determining the effect of combined drug administration in animal experiments was Burgi method (Burgi Y. Pharmacology; Drug actions and reactions.Cancerres.1978, 38(2), 284-285). The Burgi method was modified by Zhengjun JIN (Zhengjun JIN, Equal probability and curve and "Q50", Journal of the Second Shanghai Medical College; 1981, 1, 75-86) with q calculated as: q = E_{A+B}/(E_{A}+E_{B}-E_{A}×E_{B}). In the following examples of the present invention, the co-administration pharmaceutical efficacies of drug A and drug B combination administration was determined by ratio q of actual pharmaceutical efficacy /expected pharmaceutical efficacy according to Zhengjun JIN method, as follows.

When the composition group of drug A and drug B did not show meaningful pharmaceutical efficacy (e.g., r or r' ≦ 15 %), the combination administration did not show a meaningful co-administration effect either and was regarded as a negligible combination effect in the present invention. When the composition group showed meaningful pharmaceutical efficacy (e.g. tumor inhibition rate ≧ 15%), if the ratio q of actual pharmaceutical efficacy/expected pharmaceutical efficacy =1.00, then the co-administration pharmaceutical efficacy of the composition was additive (the actual effect was consistent to that of theoretical purely additive expectation); if the ratio q of actual pharmaceutical efficacy /expected pharmaceutical efficacy >1.00, then the co-administration pharmaceutical efficacy of the composition as significantly synergistic pharmaceutical efficacy (the actual effect exceeded theoretical purely additive expectation); if the ratio q of actual pharmaceutical efficacy /expected pharmaceutical efficacy <1.00, then the co-administration pharmaceutical efficacy of the composition was significantly antagonistic pharmaceutical efficacy (the actual effect was less than the theoretical purely additive expectation).

### Example 1: Preparation of pharmaceutical compositions

Numerous different local pharmaceutical compositions of the present invention can be formulated in accordance with the preparation method of the pharmaceutical compositions of the present invention described above. Several preparation examples of the pharmaceutical compositions of the present invention are set forth below.

### 1. Preparation of liquid injection formulation

The essential components of the pharmaceutical composition (e.g., 3 g sodium hydroxide as basifying agent, 7 g sodium bicarbonate as pH neutralizing agent), optionally other components, and a liquid carrier (for example, water for injection) for adjusting to a constant total volume (for example, 100 ml) were measured and taken according to a required concentration. The mixture was slowly mixed evenly, sterilized and filtered, and then divided into a required amount (for example, 10 ml/vial) and stored for use. The preparation (for example, 7% sodium bicarbonate/3% sodium hydroxide aqueous solution) can be administered locally as a liquid drug.

### 2. Preparation of an injectable powder preparation

The essential components of the pharmaceutical composition (e.g., 3 g sodium hydroxide as basifying agent, 7 g sodium bicarbonate as pH neutralizing agent), optionally existent other components and a liquid carrier (for example water for injection) for adjusting to a constant total volume (for example 100ml) were measured and taken according to a required concentration, mixed slowly and evenly, sterilized and filtered, and then divided into a required amount (e.g. 10ml/vial) for freeze-drying, stoppering and capping, to be prepared into sterile dry powder for later use.

A sterile powder in a required amount (for example, 1 vial of the above dry powder) were reconstituted in a sterile liquid in a required amount (for example water for injection) according to a required concentration of each ingredient into a desired reconstituted solution (for example, 7% sodium bicarbonate/3% sodium hydroxide aqueous solution) before it was used as a liquid drug for local administration.

### 3. Preparation of a liquid for external use

The essential components of the pharmaceutical composition (e.g., 3 g sodium hydroxide as basifying agent, 7 g sodium bicarbonate as pH neutralizing agent), optionally existent other components and a liquid carrier (for example water for injection) for adjusting to a constant total volume (for example 100ml) were measured and taken according to a required concentration, and mixed slowly and evenly. The preparation (for example, 7% sodium bicarbonate/3% sodium hydroxide aqueous solution) can be directly used as a local liquid drug for local administration.

### 4. Preparation of an atomizing preparation

The essential components of the pharmaceutical composition (e.g., 3 g sodium hydroxide, 7 g sodium bicarbonate), and the following auxiliary materials for the atomizing preparation: glycerol (2.5 g), polysorbate-80 (1.5 g), benzalkonium chloride (0.02 g), microcrystalline cellulose-sodium carboxymethyl cellulose (1.5 g), and a solvent (such as water for injection) for adjusting to a constant total volume (such as 100ml) were measured and taken according to a required concentration (as described in Table 1), and mixed slowly and evenly for later use. The preparation (for example, 7% sodium bicarbonate/3% sodium hydroxide aqueous solution) can be used as a stock solution of a spray, after being added to a sprayer, may be directly sprayed on a target region to form a liquid drug.

### Example 2: Synergistic study of compositions

In a series of experiments, the tumor inhibition effect, irritation and tissue necrosis of the drugs were studied respectively.

In the tumor inhibition effect study, successfully modeled experimental animals (mice bearing sarcoma S180 cell, with an average tumor volume of 314 mm³) were randomly divided into 2 negative control groups (01, 02) and 9 drug study groups (1-9). The negative control groups were given normal saline, and study drugs were as shown in the table below. The drugs were all in aqueous solutions, which were formulated according to the preparation method of Example 1. The negative control and study drugs were administered by intraperitoneal injection (groups 02, 6 and 9) and intratumoral injection (other study groups), respectively. Each group was administered once every 3 days for a total of 3 times, with an injection volume of 150µl/mouse. On the 5th day after the administration was ended, the animals were euthanized, and the tumor weight was measured after anatomy to the animals. The tumor inhibition rate was calculated from the negative control group of each drug administration mode. The results are shown in the following table.

In the stimulation study test, 30 mice, with no limitation to male and female, were randomly divided into 5 groups (groups 1, 3, 4, 7, and 8 in the table below) with 6 mice in each group. The experimental animals in each group were injected with 100 ul of the study drug shown in the table below into the quadriceps muscle of the right leg. After 24 h of injection, the animals were euthanized and the degrees of congestion, edema, degeneration, and necrosis at the injection site were observed for stimulus response scoring. Scores were calculated, based on the scoring criteria of 0 score for the phenomenon in the negative control group, and 5 score for the phenomenon in the 3% NaOH group. The results of the stimulus response scores were shown in the following table.

In the tissue necrosis study experiment, 30 New Zealand Large White rabbits, weighing 2.0-2.5 kg, with no limitation to male and female, were randomly divided into 5 groups (groups 1, 3, 4, 7 and 8 in the table below), with six rabbits in each group. The rabbits were first anesthetized by intravenous injection of an anesthetic agent at the ear margin, then fixed in a supine position on a rabbit stand, and the liver was exposed by a small incision on the right side of the abdomen under aseptic conditions, then a 22 G PTC needle was punctured into the center of the right lobe of the liver, the thickest part of the liver, with the tip of the needle at a depth of 1.5 cm from the Glisson's capsule of the puncture point, and the study drugs were injected slowly at a uniform speed, respectively. One intrahepatic injection was performed for each rabbit, and 1.0 mL of the study drug shown in the table below was injected. The rabbits were kept on conventional diet after drug administration, euthanized after 1 week, and the necrotic zone of the liver was determined after autopsy. The liver was incised along the center of the necrotic zone, and the extent of the necrotic zone was measured at the largest level, and the maximum and minimum diameters of the necrotic zone were measured respectively. The average diameter of the necrotic zone of liver tissue = (maximum diameter + minimum diameter)/2. The results of the average diameter of the necrotic zone of liver tissue were shown in the following table.

**Table 4**

| Gr oup s | Study drug | | Stimulus response score (m±s) | Diameter of necrotic zone of liver tissue (r±s)(cm) | Tumor inhibition effect | |
|---|---|---|---|---|---|---|
| | Composition of the drug | pH | | | Tumor weight(x±s)( g) | Tumor inhibition rate |
| 01 | normal saline | - | 0 | - | 2.21±0.32 | 0 |
| 02 | normal saline | - | - | - | 2.38±0.29 | 0 |
| 1 | 3% NaOH | 13.8 | 5.0±0.6 | 1.8±0.5 | 1.04±0.21 | 53% |
| 2 | 0.3% NaOH | 13.3 | 3.1±0.3 | 1.3±0.3 | 1.52±0.22 | 31% |
| 3 | 7% sodium bicarbonate | 8.1 | - | - | 1.59±0.20 | 28% |
| 4 | 7% sodium bicarbonate/3% NaOH | 10.2 | 2.8±0.4 | 1.1±0.3 | 0.71±0.16 | 68% |
| 5 | 0.7% sodium | 10.2 | - | - | 1.50±0.23 | 32% |
| | bicarbonate/0.3% NaOH | | | | | |
| 6 | 7% sodium bicarbonate/3% NaOH | 10.2 | - | - | 2.31±0.28 | 3% |
| 7 | 15% arginine | 10.9 | 0.5±0.1 | 0.2±0.1 | 2.01±0.24 | 23% |
| 8 | 15% arginine/7% sodium bicarbonate/3% NaOH | 10.6 | 2.5±0.3 | 1.0±0.3 | 0.29±0.11 | 87% |
| 9 | 15% arginine/7% sodium bicarbonate/3% NaOH | 10.6 | - | - | 2.33±0.31 | 2% |

It is generally believed that the antitumor efficacy of the same composition in the B-technical solution exceeds the expected limit (typically <150%, or a change from no effect to an effective effect, or a statistically significant difference in tumor weight) of pharmacodynamic improvement in the A-technical solution, it is likely to be different pharmacological drug effects.

From the results of the tumor inhibition effect in the above table, in the intraperitoneal injection series, the differences in tumor weight between each of the composition study groups 6 and 9 and the negative control group (group 02) were not statistically significant (all p>0.05), and their tumor inhibition rates were lower than the conventional effective tumor inhibition criteria (40%). Unexpectedly, using the same composition, the tumor inhibition rate was more than 20-fold higher in study group 4 as compared to study group 6, and more than 40-fold higher in study group 8 as compared to study group 9. Thus, different administration modes of the same composition showed significantly different targeting and pharmacology by completely different drug effects. The composition with same administration constitution after administration by different modes (intraperitoneal injection and local administration) might be completely different in terms of target region (local lesion region) constitution. When the intraperitoneally injected composition entered the bloodstream and then reached the local lesion region, its administration constitution were completely diluted or even dissipated by the blood (e.g., different retention of different constitution in certain organs). While the target region constitution of a locally administered composition were the same as that of its administration constitution (at least for a period of time). In other words, compositions administered by different administration modes should have different constitution characteristics in order to obtain synergistic effects.

In the prior art, bases and acids (e.g., sodium hydroxide, sodium carbonate, sodium bicarbonate, acetic acid, hydrochloric acid) administered locally above a certain concentration threshold can be used as chemical ablative agents. The bipolarity of the pH of chemical ablative agents (tending to 0 or tending to 14) is considered to be a positively correlate with their chemical ablative effect. Thus, the pH of conventional chemical ablative agents is usually chosen to be ≤ 2% (e.g., 50% acetic acid, 25% hydrochloric acid), or ≥11.5% (e.g., 7% sodium hydroxide). Under such extreme pH conditions, intense undifferentiated tissue destruction is believed to be the basis for the pharmacological action of conventional chemical ablative agents. It is generally accepted that the greater the destruction of a normal tissue by a chemical ablative agent, the greater the destruction of a diseased tissue. Many chemical ablative agents are screened by the destruction of a normal tissue (e.g., liver tissue). In the table above, the results of single drug study groups 1 and 2 were consistent with what was known in the art: the higher the stimulus response score, the larger the diameter of the necrotic zones of the liver tissue, and the higher the tumor inhibition effect.

Thus, the addition of a pH-neutralizing agent that neutralizes the pH of an acidifying agent or a basifying agent should show an antagonistic, rather than synergistic, effect on tissue destruction. Addition of 7% sodium bicarbonate (pH 6.9) decreased the pH of 3% sodium hydroxide (pH 11.8) by 1.6, and the composition was significantly neutralized (7% sodium bicarbonate/3% NaOH, pH 10.2). Compared with study group 1, study group 4 had stimulus response score and hepatic tissue necrotic zone diameter indeed decreased as predicted, and the composition group showed antagonism of local stimulus and antagonism of normal tissue destruction. However, in the above table, compared with study group 1, study group 4 showed a tumor inhibition rate greater than study group 1. Among study groups 4, 3, and 1, composition group 4 had q=1.11, which was >1.00, and the differences in residual tumor weight between group 4 and each of groups 3 and 1 respectively were statistically significant (all p<0.05), indicating a significant synergistic drug efficacy. These results suggested that addition of pH more neutral substances might allow the composition to show higher tissue disruption specificity, thereby reducing the risk of local irritation and showing synergistic safety, and even providing synergistic drug efficacy at the same time.

The emergence of this phenomenon that is not quite in line with the usual view (compositions in which the pH of the acidifying or basifying agents tended to be neutralized showed synergistic, or even significantly synergistic, pharmaceutical efficacy against lesion tissues) clearly requires a less conventional technical solution. In the above table, in the local administration series, the tumor inhibition rate of the composition study group 5 was only 47% of that of the composition study group 4 (32% vs. 68%), and the difference in tumor weight between the two groups was statistically significant (p<0.05), despite of the same component amount ratio of each composition. Between study groups 5 and 2, their tumor inhibition rates did not reach the generic standard of 40% and the difference in remaining tumor weight was not statistically significant (p>0.05), thus did not show synergistic effects. Study groups 5 and 4 used the same components and their amount ratios in the composition, but different concentrations of the acidifying agent or basifying agent and of respective pH neutralizing agents thereof, resulted in different co-administration effects. These results further suggested that, to obtain synergistic drug efficacy, compositions administered in different modes might require completely different component elements. While systemically administered compositions usually require component amount ratios only (components are intended to enter the bloodstream anyway for dilution), locally administered compositions obviously cannot be given only component amount ratios, but must also be given other characteristics (e.g., concentration).

Furthermore, in the results of stimulus response and tissue necrosis in the table above, 15% arginine/7% sodium bicarbonate/3% NaOH showed a slight increase in pH compared to 7% sodium bicarbonate/3% NaOH, yet the addition of 15% arginine resulted in decrease of stimulus response score and liver tissue necrosis zone diameter, showing local stimulus antagonism and normal tissue destruction antagonism. However, among study groups 8, 7, and 4, the composition group 8 had q= 1.15, which was >1.00, and the differences in residual tumor weight between group 8 and each of groups 7 and 4 respectively were statistically significant (all p<0.05), thus showing a significant synergistic drug efficacy. These results suggested that the addition of new local synergists might allow the composition to show higher drug efficacy, or even synergistic effect, by changing the properties of the composition (perhaps for example by increasing the buffer capacity).

In summary, the compositions of the present invention appear to exhibit a significantly different pharmacology when administered locally than that does when administered systemically. For example, the main pharmacology is not distributed in the molecular morphology into the bloodstream to attack pathogens in lesion tissues (e.g., tumor cells within tumor body), but distributed in a drug fluid form into the tissue interstitial spaces at the site of administration to attack lesion tissues (e.g., intratumor tissues).

On the basis of the above studies and further similar studies, it can be concluded that the essential conditions for the use technical solutions of the compositions of the present invention as local active ingredients in manufacturing local medicament for the treatment of local lesion diseases are:
The constitution of the pharmaceutical composition of the present invention should be such that they can be administered locally and above a concentration threshold, at which the component amount ratios of the pharmaceutical composition produce a synergistic effect. Specifically, the concentration of an acidifying agent or a basifying agent in the pharmaceutical composition should be less than its effective ablative concentration (e.g., such that the tumor inhibition concentration in tumor-bearing mice is ≥40%) but greater than the synergistic drug efficacy concentration threshold (e.g., ≥ 0.5% for a strong base or a strong acid, and ≥ 2.5% for a weak base or a weak acid). And the concentration of the pH neutralizing agent is such that allows the pharmaceutical composition tend to neutralize and the absolute value of the difference in pH between it and a single drug with the same concentration of the acidifying agent or the basifying agent be ≥ 0.25.

In addition, the composition showing synergistic drug efficacy is also determined to be a buffer solution with a buffer capacity of >0.01mol·L⁻¹ ·pH⁻¹.

In addition, the compositions of the present invention can be further increased in drug efficacy, even in synergistical drug efficacy, by the addition of local synergists.

The following Examples further investigated the technical solutions of the composition of the present invention under essential conditions described above.

### Example 3: Study on the synergistic technical solution of composition of strong base/pH neutralizing agent of the present invention

In an experiment, the test animal was a BALB/c mouse, the modeling cells were breast cancer 4T1 cells, and the transplantation tumor was modeled at 1×10⁶ cells/mouse under the skin of the animal's right axilla. Successfully modeled experimental animals (with an average tumor volume of 325 mm³) were randomly divided into 1 negative control group (group 0) and 11 drug study groups (groups 1-11). The negative control was normal saline, and the study drugs were shown in the table below. The drugs were all aqueous solutions, which were formulated according to the preparation method of Example 1. Each group was administered by intratumoral injection once every 3 days for a total of 3 times, each time at an injection dose of 150 µl//mouse. On the 5th day after the administration was ended, the animals were euthanized, and the tumor weight was determined after anatomy to the animals. The tumor inhibition rate was calculated from the negative control group. The results were shown in the following table.

**Table 5**

| Gr oup s | Drugs | | Tumor weight (x±s) | Tumor inhibition rate |
|---|---|---|---|---|
| | Composition | pH | | |
| | | | (g) | |
| 0 | normal saline | - | 2.43±0.37 | 0 |
| 1 | 5% NaOH | 14.0 | 0.28±0.12 | 72% |
| 2 | 3% NaOH | 13.7 | 1.09±0.25 | 55% |
| 3 | 1% NaOH | 13.4 | 1.41±0.23 | 42% |
| 4 | 10% sodium bicarbonate | 8.1 | 1.65±0.29 | 32% |
| 5 | 7% sodium bicarbonate | 8.1 | 1.73±0.27 | 29% |
| 6 | 4% sodium bicarbonate | 8.2 | 1.87±0.22 | 23% |
| 7 | 4% sodium bicarbonate/5% NaOH | 11.4 | 0.66±0.18 | 73% |
| 8 | 7% sodium bicarbonate/3% NaOH | 10.2 | 0.70±0.16 | 71% |
| 9 | 10% sodium bicarbonate/1% NaOH | 9.2 | 1.46±0.27 | 40% |
| 10 | 2% KCl | - | 2.19±0.31 | 11% |
| 11 | 2% KCl/1% NaOH | 11.0 | 0.85±0.16 | 65% |

In the above table, among study groups 7, 1 and 6, composition group 7 had q<1.0, showing no synergistic drug efficacy. Among study groups 8, 2, and 5, composition group 8 had q=1.04, which was >1.00, showing significant synergistic effect. Among study groups 9, 3, and 4, composition group 9 had q<1.0, showing no synergistic drug efficacy. Among study groups 11, 3 and 10, composition group 11 had q=1.42, which was >1.00, showing significant synergistic drug efficacy.

Based on the above studies and more similar studies, the preferred conditions for the pharmacodynamic synergy and safety synergy of the compositions of strong base/pH neutralizing agent of the present invention are as follows.
1. Preferable co-administration agent in the composition: the strong base comprises an alkali metal hydroxide; the pH neutralizing agent is a compound preferably selected from compounds whose pH in aqueous solution is lower than the pH of the strong base but greater than 2.0, such as selected from one or more of the following: an alkali inorganic salt of polybasic weak acid, an alkali metal salt of weak organic acid, other weak bases , salts compounded with strong acid and strong base , an acid inorganic salt of polybasic weak acid, a weak acid.
2. Chemical component characteristics of the composition (concentration and amount ratio of compounds): when the strong base is selected from alkali metal hydroxide, the concentration of the alkali metal hydroxide is ≥ 0.5%, preferably 0.75%-7.5% or 0.75%-5%; the concentration of the pH neutralizing agent is ≥1%, preferably 1%-35%; the amount ratio of the alkali metal hydroxide to the pH neutralizing agent (w _{alkali metalhydroxide}: wpH _{neutralizing agent}) is <1, preferably 1/20-1/1.25.
3. Ionic component characteristics of the composition (H⁺ concentration index): the pH difference between a single drug having the same concentration of the strong base and the composition is ≥ 0.25, preferably 0.5-3.5 or 1.0-3.5; the pH of an aqueous solution of the composition is 10.0 ± 2.0, preferably 10.0 ± 1.0.

In addition, as shown in Example 2, compositions of the strong base/pH neutralizing agent of the present invention can be further increased in drug efficacy, even in synergistical drug efficacy, by the addition of local synergists.

### Example 4: Study on the synergistic technical solution of composition of weak base/pH neutralizing agent of the present invention

In another experiment, the test animal was BALB/c mice, the modeling cells were breast cancer 4T1 cells, and the transplantation tumor was modeled at 1×10⁶ cells/mouse under the skin of the animal's right axilla. Successfully modeled experimental animals (average tumor volume 307mm³) were randomly divided into 1 negative control group (group 0) and 13 drug study groups (groups 1-13). The negative control was normal saline, and the study drugs were shown in the table below. The drugs were all aqueous solutions, which were formulated according to the preparation method of Example 1. Each group was administered by intratumoral injection once every 3 days for a total of 3 times, each time at an injection dose of 150 µl/mouse. On the 5th day after the administration was ended, the animals were euthanized, and the tumor weight was measured after anatomy to the animals. The tumor inhibition rate was calculated from the negative control group. The results were shown in the following table.

**Table 6**

| Gr oup s | Drugs | | Tumor weight(x±s) (g) | Tumor inhibition rate |
|---|---|---|---|---|
| | Composition | pH | | |
| 0 | normal saline | - | 2.26±0.37 | 0 |
| 1 | 14% sodium carbonate | 10.8 | 1.47±0.24 | 35% |
| 2 | 7% sodium carbonate | 10.8 | 1.81±0.21 | 20% |
| 3 | 2.8% sodium carbonate | 10.9 | 2.10±0.27 | 7% |
| 4 | 10% sodium bicarbonate | 8.1 | 1.58±0.22 | 30% |
| 5 | 5% sodium bicarbonate | 8.2 | 1.79±0.24 | 21% |
| 6 | 2% sodium bicarbonate | 8.2 | 2.10±0.25 | 7% |
| 7 | 2% sodium bicarbonate/14% sodium carbonate | 9.9 | 1.45±0.24 | 36% |
| 8 | 10% sodium bicarbonate/14% sodium carbonate | 9.2 | 0.50±0.13 | 78% |
| 9 | 5% sodium bicarbonate/7% sodium carbonate | 9.2 | 0.63±0.12 | 72% |
| 10 | 2% sodium bicarbonate/2.8% sodium carbonate | 9.3 | 1.29±0.21 | 43% |
| 11 | 5% sodium bicarbonate/2.8% sodium carbonate | 8.6 | 1.76±0.23 | 22% |
| 12 | 15% arginine | 11.0 | 1.74±0.28 | 23% |
| 13 | 15% arginine/5% sodium bicarbonate/7% sodium carbonate | 9.8 | 0.20±0.11 | 91% |

In the above table, among study groups 7, 1 and 6, the composition group 7 had q<1.0, showing no synergistic drug efficacy. Among study groups 8, 1 and 4, the composition group 8 had q=1.43, which was >1.00, showing significant synergistic drug efficacy. Among study groups 9, 2 and 5, the composition group 9 had q=1.43, which was >1.00, showing significant synergistic drug efficacy. Similarly, the composition group 10 also showed significantly synergistic drug efficacy, but its tumor inhibition rate was only 60% of that of the composition group 9. Among study groups 11, 3 and 5, the composition group 11 had q<1.0 , showing no synergistic drug efficacy.

In addition, among study groups 13, 12 and 9, the composition group 13 had q=1.16, which was >1.00, showing significantly synergistic drug efficacy. This result again indicated that the addition of local synergists could make the composition show synergistic drug efficacy by changing the properties of the composition (perhaps for example by increasing the buffer capacity).

Based on above studies and more similar studies, the preferred conditions for the compositions of weak base/pH neutralizing agent of the present invention are as follows.
The weak base is at a concentration of ≥ 2.5%, preferably ≥ 3.0% or ≥ 5%, or of 2.5-20%, preferably≥3.0-20% or ≥5-20%;
The pH difference between a single drug of the weak base at the same concentration and the composition is ≥ 0.25, preferably 0.5-2.5; and
The pH of the composition is 9.0 ± 2.0, preferably 9.0 ± 1.3;
The pH neutralizing agent is selected from one or more of the following group(s) satisfying the above conditions: other weak bases, alkali metal salts of weak organic acid, weak acids, and strong acids. Wherein the other weak bases are those different from the weak bases used as ablation agents;
The concentration of the pH neutralizing agent is ≥ 1%, preferably 1%-35%.

When the composition of weak base/pH neutralizing agent is a composition of alkali salt of polybasic weak acid/acid salt of polybasic weak acid, the amount ratio (w:w) of the alkali salt of polybasic weak acid to the acid salt of polybasic weak acid is greater than 0.56 and less than 7, preferably 1 to 2.

In addition, the compositions of the weak base/pH neutralizing agent of the present invention can be further increased in drug efficacy, even in synergistical drug efficacy, by the addition of local synergists.

### Example 5: A synergistic technical solution study of the composition of weak acid/pH neutralizing agent of the present invention

In one experiment, the test animal was a BALB/c mouse, the modeling cells were breast cancer 4T1 cells, and the transplantation tumor was modeled at 1×10⁶ cells/mouse under the skin of the animal's right axilla. Successfully modeled experimental animals (average tumor volume 316 mm³) were divided into 1 negative control group (group 0) and 12 drug study groups (groups 1-12). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 µl//mouse. On the 5th day after the administration was ended, the animals were euthanized, and the tumor weight was measured after anatomy to the animals. The tumor inhibition rate was calculated from the negative control group. The results were shown in the following table.

**Table 7**

| Gr oup s | Drugs | | Tumor weight(x±s) (g) | Tumor inhibition rate |
|---|---|---|---|---|
| | Composition | pH | | |
| 0 | normal saline | - | 2.18±0.32 | 0 |
| 1 | 30% acetic acid | 2.5 | 0.41±0.11 | 81% |
| 2 | 15% acetic acid | 2.5 | 0.70±0.18 | 68% |
| 3 | 3% acetic acid | 2.6 | 1.85±0.26 | 15% |
| 4 | 30% sodium acetate | 9.4 | 1.53±0.21 | 30% |
| 5 | 10% sodium acetate | 9.3 | 1.72±0.23 | 21% |
| 6 | 4% sodium bicarbonate | 8.2 | 1.68±0.27 | 23% |
| 7 | 10% sodium acetate/30% acetic acid | 2.9 | 0.61±0.16 | 72% |
| 8 | 30% sodium acetate/15% acetic acid | 4.3 | 0.46±0.13 | 79% |
| 9 | 30% sodium acetate/3% acetic acid | 6.2 | 1.48±0.28 | 32% |
| 10 | 4% sodium bicarbonate/15% acetic acid | 3.6 | 0.48±0.14 | 78% |
| 11 | 15% glycine | 6.8 | 1.81±0.26 | 17% |
| 12 | 15% glycine/4% sodium bicarbonate/15% acetic acid | 4.0 | 0.31±0.12 | 86% |

In the above table, among study groups 7, 1 and 5, the composition group 7 had q<1.00, showing no synergistic drug efficacy. Among study groups 8, 2 and 4, the composition group 8 had q>1.00, showing significant synergistic drug efficacy. Among study groups 9, 3 and 4, the composition group 9 had q<1.00, showing no synergistic drug efficacy. Among study groups 10, 2 and 6, the composition group 10 had q>1.00, showing significant synergistic drug efficacy.

Furthermore, among study groups 12, 10 and 11, the composition group 12 had q>1.00, showing significant synergistic drug efficacy. This result again illustrated that addition of local synergists can make the compositions show synergistic effect by changing the composition properties (perhaps for example by increasing the buffer capacity) with little pH change.

Based on the above studies and more similar studies, the preferred conditions for the pharmacodynamic synergy and safety synergy of the compositions of weak acid/pH neutralizing agent of the present invention are as follows.
1. The preferable active components: the weak acid is preferably selected from acetic acid and analogues thereof, such as other C1-10 aliphatic carboxylic acids with 1-3 hydroxyl substituents [hydroxy acetic acid, propionic acid, malonic acid, butyric acid, succinic acid, lactic acid (2-hydroxypropionic acid), citric acid (2-hydroxy-1,2,3-propanetricarboxylic acid), malic acid (2-hydroxy succinic acid), tartaric acid, oxalic acid, gluconic acid]; the pH neutralizing agent is preferably selected from compounds whose aqueous solution pH is higher than the pH of the weak acid, but less than 10, for example selected from one or more of the following: alkali inorganic salt of polybasic weak acid, nitrogen-containing weak base, acid inorganic salt of polybasic weak acid, alkali metal salt of weak organic acid.
2. Chemical constitution characteristics of the composition (concentration and amount ratio of a co-administration substance): the concentration of the weak acid is >3%, preferably 5-25%; the concentration of the pH neutralizing agent is ≥3%, preferably 3%-35%; amount ratio of the weak acid to the pH neutralizing agent (w_{weak acid} : w_{pH neutralizing agent}) was (5-25)/(3-35).
3. Ionic constitution characteristics of the composition (H+ concentration index): the pH difference between the composition and a single drug having the same concentration of the weak acid is ≥ 0.25, preferably 0.5-2.5; and the pH of an aqueous solution of the composition is 4.0 ± 1.5, preferably 4.0 ± 1.0.

In addition, compositions of weak acid/pH neutralizing agent of the present invention can be further increased in drug efficacy, even in synergistical drug efficacy, by addition of local synergists.

### Example 6: Study on the synergistic technical solution of composition of strong acid/pH neutralizing of the present invention

In an experiment, the test animal was a BALB/c mouse, the modeling cells were breast cancer 4T1 cells, and the transplantation tumor was modeled at 1×10⁵ cells/mouse under the skin of the animal's right axilla. Successfully modeled experimental animals (with an average tumor volume of 324 mm³) were divided into 1 negative control group (group 0) and 9 drug study groups (groups 1-9). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 µl/mouse. On the 5th day after the administration was ended, the animals were euthanized, and the tumor weight was measured after anatomy to the animals. The tumor inhibition rate was calculated from the negative control group. The results were shown in the following table.

**Table 8**

| Gr oup s | Drugs | | Tumor weight(x±s) (g) | Tumor inhibition rate |
|---|---|---|---|---|
| | Composition | pH | | |
| 0 | normal saline | - | 2.09±0.29 | 0 |
| 1 | 5% hydrochloric acid | 0.1 | 0.36±0.14 | 83% |
| 2 | 3.5% hydrochloric acid | 0.1 | 0.82±0.18 | 71% |
| 3 | 0.4% hydrochloric acid | 0.1 | 1.65±0.21 | 21% |
| 4 | 15% sodium bicarbonate | 8.1 | 1.29±0.24 | 38% |
| 5 | 5% sodium bicarbonate | 8.2 | 1.57±0.20 | 25% |
| 6 | 5% sodium bicarbonate/5% hydrochloric acid | 1.3 | 0.40±0.14 | 81% |
| 7 | 5% sodium bicarbonate/3.5% hydrochloric acid | 2.2 | 0.36±0.12 | 83% |
| 8 | 15% sodium bicarbonate/0.4% hydrochloric acid | 6.1 | 1.27±0.18 | 39% |

In the above table, among study groups 6, 1 and 5, the composition group 6 had q<1.00, showing no synergistic drug efficacy. Among study groups 7, 2 and 5, the composition group 7 had q>1.00, showing significant synergistic drug efficacy. Among study groups 8, 3 and 4, the composition group 8 had q<1.00, showing no synergistic drug efficacy.

Based on above studies and more similar studies, the preferred conditions for the compositions of strong acid /pH neutralizing agent of the present invention are as follows.
The concentration of the strong acid is >0.5%, preferably 0.75-3%;
the amount ratio of the strong acid to the pH neutralizing agent (w: w) is <1, preferably 1/20 to 1/1.25;
the pH difference between the composition and a single drug having the same concentration of weak acid is ≥ 0.25, preferably 0.5-4.0; and
the pH of the composition is 4.0 ± 1.5, preferably 4.0 ± 1.0.

The pH neutralizing agent is selected from one or more of the following group(s) satisfying the above conditions: alkali inorganic salt of polybasic weak acid, nitrogen-containing weak base, acid inorganic salt of polybasic weak acid, alkali metal salt of weak organic acid; and
the concentration of the pH neutralizing agent is ≥ 1%, preferably 1%-35%.

### Example 7: Synergistic study of compositions of the acid-base neutralization combination of the present invention and local synergists thereof

In an experiment, the test animal was a BALB/c mouse, the modeling cells were breast cancer 4T1 cells, and the transplantation tumor was modeled at 1×10⁶ cells/mouse under the skin of the animal's right axilla. Successfully modeled experimental animals (with an average tumor volume of 317 mm³) were divided into 2 negative control groups (groups 01, 02) and 20 drug study groups (groups 1-20). The negative control was normal saline, and the study drugs are shown in the table below. The drugs were all aqueous solutions, which were prepared according to the preparation method of Example 1. The negative controls and study drugs were administered intraperitoneally (groups 02, 11 and 15) and intratumorally (other study groups), respectively. All groups were dosed once every 3 days, for a total of 3 times, by intratumoral injection, with an injection volume of 150ul/mouse. On the 5th day after the administration was ended, the animals were euthanized, and the tumor weight was measured after anatomy to the animals. The tumor inhibition rate was calculated from the negative control group. The results were shown in the following table.

**Table 9**

| Gr oup s | Drugs | | Tumor weight(x±s) (g) | Tumor inhibition rate |
|---|---|---|---|---|
| | Composition | pH | | |
| 01 | normal saline | - | 2.18±0.32 | 0 |
| 02 | normal saline | - | 2.24±0.31 | 0 |
| 1 | 7% sodium bicarbonate/3% NaOH | 10.4 | 0.76±0.18 | 65% |
| 2 | 0.7% sodium bicarbonate/0.3% NaOH | 10.6 | 1.50±0.27 | 31% |
| 3 | 3% sodium bicarbonate/10% acetic acid | 3.8 | 0.85±0.16 | 61% |
| 4 | 0.3% sodium bicarbonate/1% acetic acid | 3.8 | 1.79±0.31 | 18% |
| 5 | 0.25% 5-fluorouracil | - | 1.94±0.33 | 11% |
| 6 | 1% 5-fluorouracil | - | 1.07±0.26 | 51% |
| 7 | 0.3% methylene blue | - | 2.11±0.29 | 3% |
| 8 | 1% methylene blue | - | 1.72±0.27 | 21% |
| 9 | 0.25% 5-fluorouracil/7% sodium bicarbonate/3% NaOH | 10.2 | 0.74±0.13 | 66% |
| 10 | 1% 5-fluorouracil/7% sodium bicarbonate/3% NaOH | 10.2 | 0.31±0.11 | 86% |
| 11 | 1% 5-fluorouracil/7% sodium bicarbonate/3% NaOH | 10.2 | 1.43±0.24 | 36% |
| 12 | 0.1% 5-fluorouracil/0.7% sodium bicarbonate/0.3% NaOH | 10.1 | 1.46±0.21 | 33% |
| 13 | 0.3% methylene blue/3% sodium bicarbonate/10% acetic acid | 3.8 | 0.81±0.16 | 63% |
| 14 | 1% methylene blue/3% sodium | 3.8 | 0.48±0.14 | 78% |
| | bicarbonate/10% acetic acid | | | |
| 15 | 1% methylene blue/3% sodium bicarbonate/10% acetic acid | 3.8 | 2.17±0.31 | 3% |
| 16 | 0.1% methylene blue/0.3% sodium bicarbonate/1% acetic acid | 3.8 | 1.81±0.26 | 17% |
| 17 | 15% arginine | 10.9 | 1.64±0.28 | 25% |
| 18 | 15% arginine/7% sodium bicarbonate/3% NaOH | 10.6 | 0.31±0.13 | 86% |
| 19 | 15% glycine | 6.8 | 1.81±0.26 | 17% |
| 20 | 15% glycine/3% sodium bicarbonate/10% acetic acid | 4.0 | 0.41±0.11 | 81% |

In the above table, in the intraperitoneal injection series, the tumor inhibition rates of both composition study groups 11 and 15 were lower than the conventional standard for effective tumor inhibition (40%), and the difference in tumor weight between study group 11 and the negative control group (group 02) was not statistically significant (p>0.05). Unexpectedly, using the same composition, the tumor inhibition rate was more than 2-fold higher in study group 10 than in study group 11 (86%: 36%), and more than 25-fold higher in study group 14 than in study group 14 (83%: 3%). Thus, different administration modes of the same composition showed significantly different targeting and pharmacology by completely different drug effects. The composition with same administration constitution after administration by different modes (intraperitoneal injection and local administration) might be completely different in terms of target region (local lesion region) constitution. When the intraperitoneally injected composition entered the bloodstream and then reached the local lesion region, its administration constitution were completely diluted or even dissipated by the blood (e.g., different retention of different constitution in certain organs). And, the target region constitution of a locally administered composition was the same as that of its administration constitution (at least for a period of time). In other words, compositions administered by different administration modes should have different constitution characteristics in order to obtain synergistic drug efficacies.

The emergence of this not quite usual phenomenon (i.e., substantially different effects occur between systemic administration and local administration) clearly necessitates a a less conventional technical solution. In the above table, in the local administration series, the tumor inhibition rate of the composition study group 12 was only 38% of that of the composition study group 10 (33%:86%), despite of the same component amount ratio of each composition. The tumor inhibition rate of the composition study group 16 was only 27% of that of the composition study group 14 (22%: 83%). In fact, neither the composition study group 12 nor the composition study group 16 showed a synergistic effect. However, among the study groups 10, 1 and 5, the composition group 10 had q > 1.00, showing a significant synergistic drug efficacy. Among study groups 14, 3 and 8, the composition group 14 had q>1.00, showing a significant synergistic drug efficacy. These results further suggested that, to obtain synergistic drug efficacy, compositions administered in different modes might require completely different component elements. While systemically administered compositions usually require component amount ratios only (components are intended to enter the bloodstream anyway for dilution), locally administered compositions obviously cannot be given only component amount ratios, but must also be given other characteristics (e.g., concentration).

In addition, among study groups 18, 1 and 17, the composition group 18 had q>1.00, showing a significant synergistic effect. Among study groups 20, 3 and 19, the composition group 14 had q>1.00, showing a significant synergistic effect.

In summary, the compositions of the present invention appear to exhibit a significantly different pharmacology when administered locally than that does when administered systemically. For example, its main pharmacology is not distributed in the molecular morphology into the bloodstream to attack pathogens in lesion tissues (e.g., tumor cells within tumor body), but distributed in a drug fluid form into the tissue interstitial spaces at the site of administration to attack lesion tissues (e.g., intratumor tissues).

On the basis of the above studies and further similar studies, it can be concluded that the essential conditions for the use technical solutions of the compositions of the acid-base neutralization combination and its local synergists as local active ingredients in manufacturing local medicines for the treatment of local lesion diseases were:
The constitution of the pharmaceutical composition of the present invention should be such that they can be administered locally and above a concentration threshold, at which the component amount ratios of the pharmaceutical composition produce a synergistic drug efficacy. Specifically, in the pharmaceutical composition, the concentration of the acidifying agent or the basifying agent should be ≤its effective ablative concentration (e.g., such that the tumor inhibition concentration in tumor-bearing mice experiments is ≥40%) but ≥ the synergistic drug efficacy concentration threshold (e.g., ≥ 0.5% for an acidifying agent or basifying agent concentration of a strong base or a strong acid, and ≥ 2.5% for an acidifying agent or basifying agent concentration of a weak base or a weak acid). On the basis of the acidifying agent or the basifying agent concentration, the amount ratio of each of the essential components were: the concentration of the acidifying agent or the basifying agent was as described above; the pH neutralizing agent concentration was a concentration at which it can produce a synergistic effect with the acidifying agent or the basifying agent (specifically, the concentration that allows the pharmaceutical composition tend to neutralize and the absolute value of the difference in pH between it and a single drug with the same concentration of the acidifying agent or the basifying agent be ≥ 0.25; the concentration of the local synergist is the concentration at which it can synergize with the acidifying agent or basifying agent and their respective pH-neutralizing agents (e.g. more than 5% or more than 25% of the saturated solubility of the local synergist in the composition).

In addition, the composition showing synergistic effect is also determined to be a buffer solution with a buffer capacity of >0.01mol·L⁻¹ ·pH⁻¹.

Furthermore, the synergistic combination of the acidifying agent with its pH neutralizing agent, or the basifying agent with its pH neutralizing agent appears to be the basis for the synergistic action of pharmaceutical compositions of the acid-base neutralization combination/local synergist of the present invention.

### Example 8: Further study on the synergistic technical solution about compositions of the acid-base neutralization combination and its local synergists of the present invention

In an experiment, the test animal was a BALB/c mouse, the modeling cells were breast cancer 4T1 cells, and the transplantation tumor was modeled at 1×10⁵ cells/mouse under the skin of the animal's right axilla. Successfully modeled experimental animals (with an average tumor volume of 336 mm³) were divided into 1 negative control group (group 0) and 16 drug study groups (groups 1-16). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 µl//mouse. On the 5th day after the administration was ended, the animals were euthanized, and the tumor weight was measured after anatomy to the animals. The tumor inhibition rate was calculated from the negative control group. The results were shown in the following table.

**Table 10**

| Gro ups | Study drug | | Tumor weight(x±s) (g) | Tumor inhibition rate |
|---|---|---|---|---|
| | Acid-base neutralization combination | Local synergist | | |
| 0 | (normal saline) | | 2.25±0.24 | 0 |
| 1 | 7% sodium bicarbonate/3% sodium hydroxide | | 0.79±0.17 | 65% |
| 2 | 4% sodium bicarbonate/7% sodium carbonate | | 0.72±0.15 | 68% |
| 3 | 4% sodium bicarbonate/15% acetic acid | | 0.88±0.12 | 61% |
| 4 | 4% sodium bicarbonate/2% hydrochloric acid | | 0.84±0.14 | 63% |
| 5 | | 1% 5-fluorouracil | 1.10±0.26 | 51% |
| 6 | | 1% 5 -fluorouracil/0. 1% Oriplatin (Oxaliplatin) | 0.97±0.18 | 57% |
| 7 | | 1% methylene blue | 1.73±0.23 | 23% |
| 8 | | 1% patent blue | 1.71±0.25 | 24% |
| 9 | | 5% quinine dihydrochloride | 1.46±0.21 | 35% |
| 10 | | 3% quinine monohydrochloride | 1.71±0.26 | 24% |
| 11 | 7% sodium bicarbonate/3% sodium hydroxide/1% 5-fluorouracil | | 0.36±0.11 | 84% |
| 12 | 4% sodium bicarbonate/7% sodium carbonate/1% 5-fluorouracil/0.1% Oxaliplatin | | 0.27±0.12 | 91% |
| 13 | 7% sodium bicarbonate/3% sodium hydroxide/1% methylene blue | | 0.43±0.12 | 81% |
| 14 | 7% sodium bicarbonate/3% sodium hydroxide/1% patent blue | | 0.54±0.15 | 76% |
| 15 | 4% sodium bicarbonate/15% acetic acid/5% quinine dihydrochloride | | 0.43±0.13 | 81% |
| 16 | 4% sodium bicarbonate/2% hydrochloric acid/3% quinine monohydrochloride | | 0.56±0.16 | 75% |

In the above table, among study groups 11, 1 and 5, the composition group 11 had q>1.00 , showing a significant synergistic drug efficacy. Among study groups 12, 2 and 6, composition group 12 had q>1.00 , showing a significant synergistic drug efficacy. Among study groups 13, 1 and 7, the composition group 13 had q>1.00 , showing a significant synergistic drug efficacy. Among study groups14, 1 and 8, the composition group 14 had q>1.00, showing a significant synergistic drug efficacy. Among study groups15, 3 and 9, the composition group 15 had q=>1.00 , showing a significant synergistic drug efficacy. Among study groups16, 3 and 10, the composition group 16 had q>1.00, showing a significant synergistic drug efficacy.

### Example 9: Further antitumor uses of the pharmaceutical compositions of the present invention

In this series of experiments, successfully modeled nude mice bearing human cancer cells were randomly divided into 1 negative control group and 6 study groups. The corresponding negative control was normal saline, and the 6 study drugs were: A (4% sodium bicarbonate/7% sodium carbonate), B (4% sodium bicarbonate/15% acetic acid), C (7% sodium carbonate/1% potassium hydroxide), D (15% arginine/7% sodium bicarbonate/3% sodium hydroxide), E (2% KCl/1% NaOH/20% arginine/20% xylitol), F ( 20% glycine/3% sodium bicarbonate/10% acetic acid). The drugs were all aqueous solutions, which were prepared according to the preparation method of Example 1. Each group was injected intratumorally, once every 3 days, for a total of 3 times, each time with 150µl/mouse. On the fifth day after the administration was ended, the animals were euthanized, the tumor weight was measured after anatomy, and the tumor inhibition rate was calculated from the respective negative control group.

### 1) Use in the treatment of breast tumor

In this research experiment, successfully modeled nude mice bearing human breast cancer cells (MDA-MB231) (with an average tumor volume of 303mm³) were randomly divided into 1 negative control group and 6 study groups (groups A, B, C, D, E and F). The tumor inhibition rates of groups A, B, C, D, E, and F were 79%, 73%, 77%, 92%, 97%, and 91%, respectively, which all met the generally acknowledged effective anti-tumor standard (tumor inhibition rate ≥40%).

### 2) Use in the treatment of lung tumor

In this research experiment, successfully modeled nude mice bearing human lung cancer cells (A549) (with an average tumor volume of 326mm³) were randomly divided into 1 negative control group and 6 study groups (groups A, B, C, D, E and F). The tumor inhibition rates of groups A, B, C, D, E and F were 71%, 76%, 79%,92%, 95%, and 89%, respectively, which all met the generally considered effective anti-tumor standard (tumor inhibition rate ≥40%).

### 3) Use in the treatment of thyroid tumor

In this research experiment, successfully modeled nude mice bearing human thyroid cancer cells (SW579) (with an average tumor volume of 341mm³) were randomly divided into 1 negative control group and 6 study groups (groups A, B, C, D, E and F). The tumor inhibition rates of groups A, B, C, D, E, and F were 79%, 75%, 71%,92%, 97%, and 88%, respectively, which all met the generally considered effective anti-tumor standard (tumor inhibition rate ≥40%).

### 4) Use in the treatment of prostate tumor

In this research experiment, successfully modeled nude mice bearing human prostate cancer cells (LNCaP/AR) (with an average tumor volume of 348mm³) were randomly divided into a negative control group and 6 study groups (groups A, B, C, D, E and F). The tumor inhibition rates of groups A, B, C, D, E, and F were 71%, 78%, 76%,93%, 97%, and 92%, respectively, which all met the generally considered effective anti-tumor standard (tumor inhibition rate ≥40%).

### 5) Use in the treatment of liver tumor

In this research experiment, successfully modeled nude mice bearing human liver cancer cells (HepG2) (with an average tumor volume of 309mm³) were randomly divided into a negative control group and 6 study groups (groups A, B, C, D, E and F). The tumor inhibition rates of groups A, B, C, D, E, and F were 73%, 71%, 76%,92%, 95%, and 89%, respectively, which all met the generally considered effective anti-tumor standard (tumor inhibition rate ≥40%).

### 6) Use in the treatment of head and neck tumors

In this research experiment, successfully modeled nude mice bearing human head and neck cancer cells (Fµda) (with an average tumor volume of 305mm³) were randomly divided into a negative control group and 6 study groups (groups A, B, C, D, E and F). The tumor inhibition rates of groups A, B, C, D, E, and F were 72%, 79%, 75%,93%, 97%, and 86%, respectively, which all met the generally considered effective anti-tumor standard (tumor inhibition rate ≥40%).

### 7) Use in the treatment of nasopharynx tumor

In this research experiment, successfully modeled nude mice bearing human nasopharynx cancer cells (CNE1) (with an average tumor volume of 327mm³) were randomly divided into a negative control group and 6 study groups (groups A, B, C, D, E and F). The tumor inhibition rates of groups A, B, C, D, E, and F were 79%, 73%, 71%,91%, 95%, and 88%, respectively, which all met the generally considered effective anti-tumor standard (tumor inhibition rate ≥40%).

### 8) Use in the treatment of stomach tumor

In this research experiment, successfully modeled nude mice bearing human stomach cancer cells (BGC823) (with an average tumor volume of 314mm³) were randomly divided into a negative control group and 6 study groups (groups A, B, C, D, E and F). The tumor inhibition rates of groups A, B, C, D, E, and F were 71%, 73%, 74%,88%, 91%, and 93%, respectively, which all met the generally considered effective anti-tumor standard (tumor inhibition rate ≥40%).

### 9) Use in the treatment of ovary tumor

In this research experiment, successfully modeled nude mice bearing human ovarian cancer cells (PA1) (with an average tumor volume of 311mm³) were randomly divided into a negative control group and 6 study groups (groups A, B, C, D, E and F). The tumor inhibition rates of groups A, B, C, D, E, and F were 75%, 76%, 71%,91%, 93%, and 85%, respectively, which all met the generally considered effective anti-tumor standard (tumor inhibition rate ≥40%).

### Example 10: Further study of the compositions of the acid-base neutralization combination and its local synergists of the present invention

The test animals were divided into two series, A and B. The animals in series A were non-modeled nude mice, randomly divided into four groups, and were injected with the drugs according to groups 1-4 in Table 11 by intramuscular injection of outer lateral muscle of right leg. Each group was administered once, at an injection volume of 100 µl//mouse. On day 3 after the administration was ended, the animals were euthanized, and the specimens from outer lateral muscle mass of right leg of the nude mice were dissected and taken out, and gross pathological analysis was performed. The muscle sections were measured for distinguishing the area of tissue destruction region in the normal muscle (incomplete necrosis) (T_{3d}) and the area of severe tissue destruction region therein (complete necrosis) (S_{3d}), and the results are shown in Table 11 below.

The animals in series B were modeled in 8-month-old mice as a middle-aged and elderly patient model, which was not consistent with the commonly used young patient model in terms of tumor formation, tumor histomorphology, and body immunocompetence. 1×10⁶ mouse liver cancer Hepa1-6 cells/mouse were injected into the right axilla subcutaneously for transplantation tumor modeling, and the mean tumor volume of successfully modeled mice was 136.6 mm³. The model animals were randomly divided into 13 groups. Each group was administered once by intratumoral injection according to the drug grouping in Table 11 with an injection volume of 100 µl/mouse. The tumor volume (V) was measured on day 3 and day 14 after drug administration, and the tumor inhibition rates (r'_{3d}, r'_{14d}) were calculated at each of time points relative to the negative control group, and the results were shown in Table 11 below. The drugs administered were prepared according to the conventional preparation method for aqueous solution or according to the preparation method in Example 1.

**Table 11 Experimental data after drug administration in different groups**

| Gro up No. | | Study drug | Series A | | Series B | |
|---|---|---|---|---|---|---|
| | Drug types | Component, dosage | S_{3d}(mm² ) | T_{3d}(mm² ) | r'_{3d} | _{r'14d} |
| 1 | negative control | normal saline | 0 | 0 | - | - |
| 2 | basifying agent | 3% sodium hydroxide aqueous solution | 18.3 | 29.6 | 31.2% | 2.8% |
| 3 | pH neutralizing agent | 7% sodium bicarbonate aqueous solution | 0 | 0 | 4.1% | 3.9% |
| 4 | acid-base neutralization combination | 3% sodium hydroxide/7% sodium bicarbonate aqueous solution | 0 | 47.6 | 48.3% | 27.1% |
| 5 | cytotoxic drug | 1% 5-fluorouracil | - | - | 26.8% | 3.1% |
| 6 | staining agent | 1% methylene blue | - | - | 27.8% | 16.9% |
| 7 | polymer blood volume expander | 2.5% dextran40 aqueous solution | - | - | 4.2% | 5.3% |
| 8 | probiotic component | 5% yeast hydroxymethyl glucan aqueous solution | - | - | 4.5% | 7.8% |
| 9 | acid-base neutralization | 3% sodium hydroxide/7% | - | - | 77.2% | 35.5% |
| | combination /cytotoxic drug | sodium bicarbonate/5-fluoro uracil | | | | |
| 10 | acid-base neutralization combination /staining agent | 3% sodium hydroxide/7% sodium bicarbonate/1% methylene blue | - | - | 60.5% | 40.9% |
| 11 | acid-base neutralization combination /polymer blood volume expander | 3% sodium hydroxide/7% sodium bicarbonate/2.5% dextran40 aqueous solution | - | - | 25.1% | 31.8% |
| 12 | acid-base neutralization combination /probiotic component | 3% sodium hydroxide/7% sodium bicarbonate/5% yeast hydroxymethyl glucan aqueous solution | - | - | 29.6% | 41.3% |
| 13 | basifying agent/cytotoxic drug | 3% sodium hydroxide/5-fluorour acil | | | 63.8% | 4.7% |

The actual/expected ratio q_{3d} for short-term drug efficacy and the actual/expected ratio q_{14d} for medium-term drug efficacy were calculated based on the drug efficacies (r'_{3d}, r'_{21d}) at different times (day 3, day 14), respectively.

In series A of the above table, S_{3d} for groups 2 and 4 were 18.3±7.52 mm², 0 mm², respectively, indicating that the basifying agent maximized the local action intensity as expected, while the pH neutralizing agent in the neutralization combination reduced the local action intensity of the basifying agent as expected. It is generally believed that the area of necrotic region should be positively correlated with the area of completely necrotic region. However, the T_{3d} for groups 2 and 4 were the opposite, which were 29.6±8.31mm², and 47.6±15.32mm², respectively, indicating that the acid-base neutralization combination produced a synergistic effect on a general tissue destruction, although it produced an antagonistic effect on a severe tissue destruction.

It is generally believed that tumor cells proliferate rapidly and intensely, and the pharmaceutical effects of tumor inhibition should be associated with the effect of a locally acting drug on the degree of complete necrosis (maximizing the local effect) in order to have effective inhibition; and the pharmaceutical efficacy of short-term tumor inhibition (r'_{3d}) should be positively correlated with the strength of the drug (in terms of severe tissue destruction). However, the r'_{3d} results from groups 2 and 4 in series B (r'₄>r'₂) were contrary to the S_{3d} results from groups 2 and 4 in series A (S₂>S₄) and consistent with the T_{3d} results (T₂<T₄), and the short-term actual/expected ratio in group 4 had q_{3d} >1.00, indicating that the synergistic effect on general tissue destruction produced by the acid-base neutralization combination could be shown as a synergistic effect on tumor tissue destruction. It is generally believed that a medium-term pharmaceutical efficacy (r'_{14d}) is a continuation of a short-term efficacy (r'_{3d}), which is certainly consistent to a short-term efficacy. However, in groups 2 and 4 of series B, the medium-term comparative result of (r'_{14d4}/r'_{14d2}=1.15) was far from their short-term comparative result (r'_{3d4}/r'_{3d2}=9.68). In addition, group 4 also showed a medium-term synergistic effect, and the actual/expected ratio of medium-term to short-term, q_{14d}/q_{3d}, was greater than 200%, indicating that the acid-base neutralization combination produced a new pharmacology that far exceeded the expected local synergistic effect.

In series B of the above table, the acid-base neutralization combination/co-administration substance in groups 9 and 10 showed a short-term synergistic effect (q_{3d}>1.00) and a medium-term synergistic effect (q_{14d}>1.00), whereas the acid-base neutralization combination/co-administration substance in groups 11 and 12 showed a short-term antagonistic effect (q_{3d}<1.00) and a medium-term synergistic effect (q_{14d}>1.00), indicating that a medium-term synergistic effect was the fundamental feature of the acid-base neutralization combination /co-administration substance. The above results indicated that the activity characteristic of the composition of acid-base neutralization combination or acid-base neutralization combination /co-administration substance of the present invention was not to provide maximum local action, but to provide maximum penetration of the appropriate local action within a tissue.

Based on the results of above examples and the results of other similar tests, the acid-base neutralization compositions of the present invention (compositions of the present invention) exceeded the expectations of acid-base acting drugs (e.g., highly concentrated acids, highly concentrated bases, etc.) in prior art (abbreviated as prior art drugs) in the following aspects.
1) its pharmacology exceeded expectations: the characteristic pharmacology of prior art drugs was effective local action on tissues, preferably maximizing local action, while the characteristic pharmacology of the compositions of the present invention was effective inter-tissue penetration, preferably maximizing penetration;
2) its pharmacological constitution exceeded expectations: in order to achieve its different pharmacology, prior art drugs required a maximum strength of local action, thus requiring a very high acid (or base) pharmacological concentration (e.g. 50% acetic acid, 14% sodium hydroxide) and its pure acid (or base) pH, whereas the compositions of the present invention did not require maximum strength of local action, but require maximum strength of penetration action, thus requiring acid-base neutralization combination, which was opposite to an acid (or base). The compositions of the present invention comprise an appropriate amount ratio (mainly to ensure sufficient neutralizing effect) of an acidifying agent to its pH adjusting agent (or of a basifying agent to its pH adjusting agent), and a smaller pharmacological concentration of the acidifying agent (or the basifying agent) (e.g. ≦ 15% acetic acid, ≦ 5% sodium hydroxide), resulting in an acid-base neutralizing solution whose pH was far enough away from that of the acid (or the base);
3) its technical effect exceeded expectations: in terms of safety, the pharmacological constitution of prior art drugs brought about a strong local irritation and a risk of acid (or base) overdose, while the pharmacological constitution of the compositions of the present invention could greatly reduce the local irritation and the risk of acid (or base) overdose. In terms of effectiveness, the penetration action of the pharmacological constitution of the compositions of the present invention could produce a greater range of effective action. The therapeutic efficacy and the safety made the compositions of the present invention more suitable than prior art drugs for middle-aged and elderly patients, preferably elderly who were at risk of local irritation and acid (or base) overdose.

Based on the results of above examples and the results of other similar tests, the compositions of an acid-base neutralization combination and its co-administration substance of the present invention (compositions of the present invention) exceeded the expectations of the expectable compositions in prior art comprising a locally acting drug (e.g., ethanol, highly concentrated acids, highly concentrated bases, etc.) and its co-administration substance (abbreviated as prior art drugs) in the following respects.
1) its pharmacology exceeded expectations: the characteristic pharmacology of the prior art composition was at most synergistic of local effects, while the characteristic pharmacology of the compositions of the present invention was synergistic effects comprising the effective inter-tissue penetration of the acid-base neutralization combination;
2) its pharmacological constitution exceeded expectations: the pharmacological constitution of the above acid-base neutralization combination having penetration effect exceeded expectations;
3) its technical effect exceeded expectations: the synergistic effect comprising the effective inter-tissue penetration effect from the acid-base neutralization combination could produce a medium-term therapeutic efficacy beyond that expected from the prior art compositions.

Some other compositions of the present invention prepared by the method of Example 1 (such as the synergistic composition in each example) can also obtain similar results in the use of treatment of the above various tumors.

The interventional treatment by local administration for a disease with local lesion symptoms, especially a refractory disease, usually takes a tumor as a model. Among diseases related to local lesions, the mechanism of tumors is extremely complex and the most difficult to treat. The local drug administration technical solution obtained by using a tumor as a model is usually also applicable to other local lesion-related diseases. The following experiments study more applications of the composition of the present invention.

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. Use of an acid-base neutralization combination as a local active ingredient in manufacturing a local pharmaceutical composition for the treatment of a local lesion disease, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralize, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

2. A local pharmaceutical composition for the treatment of a local lesion disease, comprising an acid-base neutralization combination as a local active ingredient, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the a basifying gent is selected from one or more of weak bases or strong bases; the pH neutralizing agent is selected from an acidic substance or an alkaline substance capable of making the acidifying agent or the basifying agent tend to pH neutralize, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

3. A local pharmaceutical composition for the treatment of a local lesion disease, comprising an acid-base neutralization combination as a local active ingredient and a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralize, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

4. A method for treating a local lesion disease, comprising administering to a local lesion region of an individual in need thereof a therapeutically effective amount of a pharmaceutical composition that can cause tissue necrosis in the target region, wherein the pharmaceutical composition comprises an acid-base neutralization combination capable of acting as a local active ingredient and optionally a local synergist of the neutralization combination, wherein the acid-base neutralization combination comprises an acidifying agent and a pH neutralizing agent thereof, or a basifying agent and a pH neutralizing agent thereof, and wherein the acidifying agent is selected from one or more of strong acids or weak acids; the basifying agent is selected from one or more of weak bases or strong bases; the pH neutralizing agent is selected from an acidic substance or an alkaline substance capable of making the acidifying agent or the basifying agent tend to pH neutralize, and wherein the acidifying agent, the basifying agent, and the pH neutralizing agent are preferably selected from an acidic substance or an alkaline substance capable of being used as a pH adjusting agent rather than as a conventional active component for the treatment of local lesion diseases.

5. The use, the pharmaceutical composition, or the method according to any one of claims 1-4, wherein the local active ingredient comprises an ingredient having an effective inter-tissue penetration effect, preferably a maximum inter-tissue penetration effect.

6. The use, the pharmaceutical composition, or the method according to any one of claims 1-5 , wherein the pharmaceutical composition is so prepared, composed or administered as to make the acid-base neutralization combination provide the local activity: the acidifying agent and its pH neutralizing agent, or the basifying agent and its pH neutralizing agent enter the target region at an administration amount ratio (w/w) (W_{basifying agent}/W_{neutralizing agent}, or W_{acidifying agent}/W_{neutralizing} agent) of (0.5-35)/(1-35), preferably (1.5-35)/(1-35).

7. The use, the pharmaceutical composition, or the method according to any one of claims 1-5, wherein the pharmaceutical composition is so prepared, composed or administered as to make the acid-base neutralization combination provide the local activity: the acidifying agent or the basifying agent enters the target region at an administration concentration of ≥0.5%, preferably ≥0.75% or ≥2.5%, and the pH neutralizing agent enters the target region at an administration concentration such that the administration pH value of the pharmaceutical composition is closer to neutral than the pH of a single drug having the same concentration of the acidifying agent or the basifying agent, and the absolute value of the difference between the two pHs (| pH of the pharmaceutical composition to be administered - pH of the single drug having the same concentration of the acidifying agent or the basifying agent | ) is ≥ 0.25, ≥ 0.5, or ≥1.0.

8. The use, the pharmaceutical composition, or the method according to claim 6, wherein the administration pH value of the pharmaceutical composition is 7.5±4.5, 10.0±2.0 or 4.0±2.0, preferably 8.5-11.5, 9.5-11.5, 10.0-11.0, 3.0-5.0, or 3.5-4.5.

9. The use, the pharmaceutical composition, or the method according to any one of claims 1-8, wherein:
the basifying agent is a strong base, and the pharmaceutical composition is so prepared, composed or administered as to make the acid-base neutralization combination provide the local activity: the strong base is administered at a concentration (w/v) of ≥0.5%, preferably ≥0.75% or ≥1%; of 0.5-10%, preferably 0.75-10% or 1-10%; or
the basifying agent is a weak base, and the pharmaceutical composition is so prepared, composed or administered as to make the acid-base neutralization combination provide the local activity: the weak base is administered at a concentration (w/v) of ≥2.5%, preferably ≥3.0% or ≥5%; of 2.5-35%, preferably 3.0-35% or 5-35%; or
the acidifying agent is a strong acid, and the pharmaceutical composition is so prepared, composed or administered as to make the acid-base neutralization combination provide the local activity: the strong acid is administered at a concentration (w/v) of ≥ 0.5%, preferably ≥ 0.75% or ≥ 1%; of 0.5-10%, preferably 0.75-10% or 1-10%; or
the acidifying agent is a weak acid, and the pharmaceutical composition is so prepared, composed or administered as to make the acid-base neutralization combination provide the local activity: the weak acid is administered at a concentration (w/w) of ≥2.5%, preferably ≥3.0% or ≥5%, or of 2.5-20%, preferably 3.0-20% or 5-20%.

10. The use, the pharmaceutical composition, or the method according to any one of claims 1-9, wherein the pharmaceutical composition is so prepared, composed or administered as to make the acid-base neutralization combination provide the local activity: the pH neutralizing agent is selected from a group consisting of one or more of: a weak base, a salt compounded with a strong acid and/or a strong base, an alkali metal salt of a weak organic acid, and a weak acid, and the pH neutralizing agent is administered at a concentration of ≥1%, preferably 2-35%.

11. The use, the pharmaceutical composition, or the method according to any one of claims 1-10, wherein the basifying agent is one or more of strong bases, the pH neutralizing agent is selected from a group consisting of one or more of: a weak base, a salt compounded with a strong acid and/or a strong base, an alkali metal salt of weak organic acid, and a weak acid, and the pharmaceutical composition is so prepared, composed or administered as to make the acid-base neutralization combination provide the local activity: the strong base is administered at a concentration of ≥0.5%, preferably ≥0.75% or ≥1%; of 0.5-10%, preferably 0.75-10% or 1-10%, and the pH neutralizing agent is administered at a concentration of ≥1%, preferably 2-35%.

12. The use, the pharmaceutical composition, or the method according to any one of claims 1-10, wherein the basifying agent is one or more of weak bases, the pH neutralizing agent is selected from a group consisting of one or more of: other weak bases, a salt compounded with a strong acid and/or a strong base, an alkali metal salt of weak organic acid, and a weak acid, and the pharmaceutical composition is so prepared, composed or administered as to make the acid-base neutralization combination provide the local activity: the weak base is administered at a concentration of ≥2.5%, preferably ≥3.0% or ≥5%; of 2.5-35%, preferably 3.0-35% or 5-35%, and the pH neutralizing agent is administered at a concentration of ≥1%, preferably 2-35%.

13. The use, the pharmaceutical composition, or the method according to any one of claims 1-10, wherein the acidifying agent is one or more of strong acids, the pH neutralizing agent is selected from a group consisting of one or more of: a weak base, a salt compounded with a strong acid and/or a strong base, an alkali metal salt of weak organic acid, and a weak acid; and the pharmaceutical composition is so prepared, composed or administered as to make the acid-base neutralization combination provide the local activity: the strong acid is administered at a concentration of ≥ 0.5%, preferably ≥ 0.75% or ≥ 1%; of 0.5-10%, preferably 0.75-10% or 1-10%, and the pH neutralizing agent is administered at a concentration of ≥ 1%, preferably 2-35%.

14. The use, the pharmaceutical composition, or the method according to claims 1-10, wherein the acidifying agent is one or more of weak acids, the pH neutralizing agent is selected from a group consisting of one or more of a weak base, a salt compounded with a strong acid and/or a strong base, an alkali metal salt of weak organic acid, and other weak acids; and the pharmaceutical composition is so prepared, composed or administered as to make the acid-base neutralization combination provide the local activity: the weak acid is administered at a concentration of ≥ 2.5%, preferably 2.5-20%, preferably 3.0-20% or 5-20% ≥ 0.75%, and the pH neutralizing agent is administered at a concentration of ≥ 1%, preferably 2-35%.

15. The use, the pharmaceutical composition, or the method according to any one of claims 1-14, wherein the strong base comprises an alkali metal hydroxide, wherein the alkali metal hydroxide comprises sodium hydroxide, potassium hydroxide, calcium hydroxide.

16. The use, the pharmaceutical composition, or the method according to any one of claims 1-14, wherein the weak base comprises an alkali inorganic salt of a polybasic weak acid, an acid inorganic salt of a polybasic weak acid, and a nitrogen-containing weak base.

17. The use, the pharmaceutical composition, or the method according to claim 16, wherein the alkali inorganic salt of polybasic weak acid comprises sodium phosphate, sodium carbonate, potassium carbonate, and borax.

18. The use, the pharmaceutical composition, or the method according to claim 16, wherein the acid inorganic salt of a polybasic weak acid comprises sodium dihydrogen phosphate, disodium hydrogen phosphate, sodium bicarbonate, potassium bicarbonate, calcium bicarbonate, and sodium hydrosulfate.

19. The use, the pharmaceutical composition, or the method according to claim 16, wherein the nitrogen-containing weak base is selected from a group comprising ammonia, ammonia chloride, 2-aminoethanol, trometamol, triethanolamine, trihydroxymethylaminomethane, 2-aminoethanol, trometamol, triethanolamine, meglumine, N-ethyl glucamine.

20. The use, the pharmaceutical composition, or the method according to any one of claims 1-14, wherein the weak acid is selected from one or more of an inorganic weak acid or/and an organic weak acid, wherein the inorganic weak acid comprises phosphoric acid, carbonic acid, boric acid, sulfurous acid; the organic weak acid comprises a C1-10 aliphatic carboxylic acid substituted with 1-3 hydroxyl groups, such as acetic acid, hydroxy acetic acid, propionic acid, malonic acid, butyric acid, succinic acid, lactic acid (2-hydroxypropionic acid), citric acid (2-hydroxy-1,2,3-propane-tricarboxylic acid), malic acid (2-hydroxybutanedioic acid), tartaric acid, oxalic acid, gluconic acid.

21. The use, the pharmaceutical composition, or the method according to any one of claims 1-14, wherein the strong acid comprises hydrochloric acid, sulphuric acid, nitric acid, perchloric acid, selenic acid, hydrobromic acid, hydroiodic acid.

22. The use, the pharmaceutical composition, or the method according to any one of claims 3-21, wherein the local synergist is selected from one or more of a cytotoxic drug and/or a conventional ineffective drug, and in the pharmaceutical composition:
the concentration of the cytotoxic drug is 50-100% of its solubility; and/or
the concentration of the conventional ineffective drug is 0.35-40%, preferably 1-40%.

23. The use, the pharmaceutical composition, or the method according to claim 22, wherein the cytotoxic drug is selected from a group consisting of one or more of a drug that damages the structure and function of DNA, a drug that is intercalated in DNA and interferes with transcription of RNA, a drug that interferes with DNA synthesis, and a drug that affects protein synthesis; preferably selected from a group consisting of one or more of an alkylating agent, for example cyclophosphamide, and carmustine; a metal platinum complex, for example, cisplatin, and carboplatin; a DNA topoisomerase inhibitor, for example doxorubicin, topotecan, and irinotecan; anti-tumor antibiotics, for example actinomycins, daunorubicin; a pyrimidine antagonist, for example a uracil derivative 5-fluorouracil, ftorafur, tegadifur, cytosine derivative cytarabine, cyclocytidine, 5-azacytidine; taxanes, for example paclitaxel, docetaxel.

24. The use, the pharmaceutical composition, or the method according to claim 22, wherein the conventional ineffective drug is one or more selected from a group consisting of one or more of amino acid based nutrients, carbohydrate nutrients, lipid nutrients, pigment and aromatic compounds, salicylic acid compounds, quinine compounds, blood volume expanders, probiotic components.

25. The use, the pharmaceutical composition, or the method according to claim 24, wherein the amino acid based nutrient is selected from one or more of basic amino acid based nutrients and/or non-basic amino acid based nutrients, wherein the basic amino acid based nutrient is, for example, arginine, lysine, histidine, preferably arginine; the non-basic amino acid based nutrient is, for example, a group consisting of one or more of neutral amino acids, acidic amino acids, amino acid salts, wherein the neutral amino acid is, for example, glycine, tryptophan, tyrosine, serine, cysteine, methionine, asparagine, glutamine, threonine, alanine, valine, leucine, isoleucine, phenylalanine, proline; the acidic amino acid is, for example, aspartic acid, glutamic acid; the amino acid salts comprises salts formed by an amino acid as described above with an acid, for example lysine hydrochloride, histidine hydrochloride, glutamic acid hydrochloride, cysteine hydrochloride, arginine hydrochloride, glycine sulfate, iron glycine sulfate, lysine hydrochloride, aspartic hydrochloride; and the amino acid based nutrient is administered at a concentration (w/v) of ≥ 5%, preferably 10-30%.

26. The use, the pharmaceutical composition, or the method according to claim 24, wherein the carbohydrate nutrient is selected from one or more of glucose, fructose, oligochitosan, glucosamine, lactulose, sorbitol, ribose, sorbose, mannose, galactose, sucrose, lactose, trehalose, xylo-oligosaccharide, fructooligosaccharide, mannose oligosaccharide, xylitol; and more preferably is selected from one or more of glucose, sodium gluconate, oligochitosan, glucosamine, lactulose, ribose, oligomannose, xylitol; and the carbohydrate nutrient is administered at a concentration (w/v) of ≥ 10%, preferably 10-40%.

27. The use, the pharmaceutical composition, or the method according to claim 24, wherein the lipid nutrient is selected from one or more of a vegetable oil, eicosapentaenoic acid (EPA), docosahexaenoic acid (DHA), long-chain fat emulsion, medium-chain fat emulsion, phospholipids, and the lipid nutrient is administered at a concentration (w/v) of ≥ 4%, preferably 4-25%.

28. The use, the pharmaceutical composition, or the method according to claim 24, wherein the pigment and aromatic compounds is selected from one or more: methylene blue, patent blue, isosulfan blue, Bengal red, and the pigment and aromatic compounds is administered at a concentration (w/v) of ≥ 0.35%, preferably 0.5-10%.

29. The use, the pharmaceutical composition, or the method according to claim 24, wherein the blood volume expander is selected from a group consisting of one or more of: a dextran blood volume expander, a starch derivative blood volume expander, a gelatin derivative blood volume expander, a synthetic blood volume expander, preferably a dextran blood volume expander selected from a group consisting of one or more of: dextran 10, dextran 40, dextran 70, and the blood volume expander is administered at a concentration (w/v) of ≦ 30%, preferably 2-30%, more preferably 2-5%, 5-15% or 15-25%.

30. The use, the pharmaceutical composition, or the method according to claim 24, wherein the probiotic component is selected from one or more probiotic components or derivatives comprising: a probiotic water-soluble component, a probiotic semi-fluidic component, a probiotic component water-insoluble particle, inactivated probiotics, preferably selected from one or more of: a probiotic water-soluble component, or/and a probiotic semi-fluidic component, and wherein the probiotic component or derivative is administered at a concentration (w/v) of ≥ 0.25%, preferably 0.25-25%, more preferably 0.5-5%, 5-10% or 15-25%.

31. The pharmaceutical composition, the use or the method according to any one of claims 1-30, wherein the local lesion comprises a tumor, non-neoplastic enlargement, local inflammation, abnormal secretory gland function, and a skin disease.

32. The pharmaceutical composition, the use or the method according to claim 31, wherein the tumor comprises a malignant tumor and a non-malignant tumor.

33. The pharmaceutical composition, the use or the method according to claim 32, wherein the malignant tumor comprises breast cancer, pancreatic cancer, thyroid cancer, nasopharyngeal cancer, prostate cancer, liver cancer, lung cancer, intestinal cancer, oral cancer, esophageal cancer, stomach cancer, laryngeal cancer, testicular cancer, vaginal cancer, uterine cancer, and ovarian cancer.

34. The pharmaceutical composition, the use or the method according to claim 32, wherein the non-malignant tumor comprises breast tumor, pancreatic tumor, thyroid tumor, prostate tumor, liver tumor, lung tumor, intestinal tumor, oral tumor, esophageal tumor, stomach tumor, nasopharyngeal tumor, laryngeal tumor, testicular tumor, vaginal tumor, uterine tumor, fallopian tube tumor, and ovarian tumor.

35. The pharmaceutical composition, the use or the method according to claim 31, wherein the non-neoplastic enlargement comprises hyperplasia (such as hyperplasia of breast, pancreas, thyroid, parathyroid, prostate), cysts (such as cysts of breast, thyroid, parathyroid), nodules (such as nodules in breast, thyroid, parathyroid), abnormal venous mass (such as hemorrhoids), local inflammation and swelling, microbial infection and swelling.

36. The pharmaceutical composition, the use or the method according to claim 31, wherein the local inflammation refers to non-neoplastic inflammation at a local site, including alterative inflammation, exudative inflammation, and proliferative inflammation.

37. The pharmaceutical composition, the use or the method according to claim 36, wherein the local inflammation comprises one or more of: arthritis, mastitis, pancreatitis, thyroiditis, prostatitis, hepatitis, pneumonia, enteritis, oral inflammation, pharyngitis, periodontitis, esophagitis, gastritis, gastric ulcer, rhinitis, sinusitis, laryngitis, tracheitis, bronchitis, vaginitis, metritis, salpingitis, and oophoritis.

38. The pharmaceutical composition, the use or the method according to claim 31, wherein the abnormal secretory gland function comprises hyperfunction of secretory glands (such as hyperthyroidism) and hypofunction of secretory glands (such as hypothyroidism, hypoinsulinism).

39. The pharmaceutical composition, the use or the method according to claim 31, wherein the skin disease refers to a lesion that is primary or secondary to a skin or a skin accessory organ, and includes one or more of the followings: skin cancer, non-malignant skin tumors, viral skin diseases (such as herpes, warts, rubella, hand-foot-and-mouth disease), bacterial skin diseases (such as impetigo, boils, leprosy), fungal skin diseases (such as various ringworms), sexually transmitted diseases (such as syphilis, gonorrhea, and condyloma acuminatum), allergic and autoimmune skin diseases (such as contact dermatitis, eczema, urticaria), physical skin diseases (such as solar skin diseases, frostbite, corns, cracked skin of hand and foot, pressure sores), connective tissue diseases (such as lupus erythematosus), pigmented skin diseases (such as freckles, pigmented moles, various spots), skin appendage diseases (such as acne, rosacea, seborrheic dermatitis, alopecia areata, baldness, hyperhidrosis and bromhidrosis).

40. A device for treating a local lesion disease comprising the pharmaceutical composition according to any one of claims 2-39.

41. The use, the pharmaceutical composition, or the method according to any one of claims 1-14, wherein the alkali metal salt of a weak organic acid comprises potassium hydrogen phthalate, sodium acetate, sodium propionate, sodium butyrate, sodium malonate, sodium lactate, sodium citrate, sodium 2-hydroxypropane-1,2,3-tricarboxylate, sodium malate, sodium dodecyl sulfate.

42. The use, the pharmaceutical composition, or the method according to any one of claims 1-14, wherein the salt compounded with a strong acid and a strong base comprises sodium chloride, potassium chloride, sodium iodide, potassium iodide.
